# EUROPEAN PATENT APPLICATION

(11) **EP 4 767 932 A1**
(43) Date of publication of application: **01.07.2026**
(21) Application number: 24223454.0
(22) Date of filing: 27.12.2024
(51) Int. Cl.: A61B 5/145

(54) **ANALYTE SENSOR BASED ON MONITORING MULTIPLE ANALYTES**

(71) Applicant: Syai UK Ltd, Cambridge CB2 8DL (GB)
(72) Inventor: WANG, Zhihua, Cambridge, CB2 8DL (GB)
(74) Representative: Nederlandsch Octrooibureau

(57) **Abstract**

The present application provides an analyte sensor based on monitoring multiple analytes. The analyte sensor includes a channel structure, and the analyte sensor can be implanted into a human body for simultaneously monitoring multiple analytes, and has the characteristics of good flexibility, small size, coexisting multiple analysis methods, low manufacturing cost, simple process flow, and long-term monitoring. The analyte sensor is configured to simultaneously monitor analytes/indicators, and can achieve the characteristics of two functional areas in one plane at the same time. The introduction of the channel structure of the present application makes the thickness of the analyte sensor more suitable and the wearing experience better. The introduction of the channel structure can also avoid problems such as unstable signal transmission caused by small electrode area or narrow electrode cables, resulting in poor sensor stability.

## Description

### Technical Field

The present application belongs to the technical field of sensors, and specifically relates to an analyte sensor based on monitoring multiple analytes.

### Background

Flexible electrodes refer to electrodes manufactured using flexible substrates. Biosensors made of flexible electrodes have the characteristics of light weight, high flexibility, stretchability, and excellent comfort. Biosensors made of flexible electrodes are widely used in testing of nucleic acids, proteins, glucose, bacteria, toxins, cells, and pesticides in vitro.

At present, the vast majority of flexible electrodes are used for temporary in vitro testing. With the increasing demand, there is a growing need for sensors that can continuously monitor analytes in vivo. However, the current size of flexible electrodes is relatively large, which cannot meet the adaptability of the human body to sensors. At the same time, the complex manufacturing process flow and high manufacturing costs of sensors further limit the development of analyte sensors.

### Summary

In order to improve the shortcomings of the prior art, the present application provides an analyte sensor based on monitoring multiple analytes. The analyte sensor can be implanted into a human body for simultaneously monitoring multiple analytes, and has the characteristics of good flexibility, small size, multiple analysis methods, low manufacturing cost, simple process flow, and long-term monitoring.

The purpose of the present application is achieved through the following technical solution:
An analyte sensor, characterized in that, the analyte sensor comprises a substrate, a first working electrode, a second working electrode, a reference electrode, a counter electrode, a first sensing layer and a second sensing layer; the first sensing layer is provided on the first working electrode, and the second sensing layer is provided on the second working electrode; any two of the first working electrode, the second working electrode, the reference electrode and the counter electrode are located on a first surface of the substrate, and the remaining two electrodes of the first working electrode, the second working electrode, the reference electrode and the counter electrode are located on a second surface of the substrate opposite to the first surface ; the two electrodes located on the first surface of the substrate are spatially separated by a channel, and/or, the two electrodes located on the second surface of the substrate opposite to the first surface are spatially separated by a channel; the width of the channel is 1µm -280µm; the length of the channel is 30µm-10,000µm.

An analyte sensor, characterized in that, the analyte sensor comprises a substrate, a first working electrode, a second working electrode, a reference electrode, a counter electrode, a first sensing layer and a second sensing layer; the first sensing layer is provided on the first working electrode, and the second sensing layer is provided on the second working electrode; any three of the first working electrode, the second working electrode, the reference electrode, and the counter electrode are located on a first surface of the substrate, and the remaining one electrode of the first working electrode, the second working electrode, the reference electrode, and the counter electrode is located on a second surface of the substrate opposite to the first surface; one of the three electrodes located on the first surface of the substrate is provided on the surface of the substrate, and the remaining two electrodes are stacked on the surface of the one electrode and separated from the one electrode by a dielectric layer; the remaining two electrodes are spatially separated by a channel; the width of the channel is 1µm-280µm; the length of the channel is 30µm-10,000µm.

The beneficial effects of the present invention are as follows:
The present application provides an analyte sensor based on monitoring multiple analytes. The analyte sensor includes a channel structure, and the analyte sensor can be implanted into a human body for simultaneously monitoring multiple analytes, and has the characteristics of good flexibility, small size, multiple analysis methods, low manufacturing cost, simple process flow, and long-term monitoring. The analyte sensor has is configured to simultaneously monitor analytes/indicators, and the analyte sensor has the function of coexisting multiple analysis methods; the analyte sensor has the function of implantable and continuous monitoring, low manufacturing cost, simple process flow, small size, and good flexibility; the analyte sensor can simultaneously achieve the characteristics of two functional areas in one plane. The introduction of the channel structure of the present application makes the thickness of the analyte sensor more suitable and the wearing experience better. The introduction of the channel structure can also avoid problems such as unstable signal transmission caused by small electrode area or narrow electrode cables, resulting in poor sensor stability.

### Brief Description of the Drawings

FIG. 1 is a view of the sensor electrode structure A in Embodiment 1.
FIG. 2 is a structural diagram of the section A-A' of the sensor electrode structure A in Embodiment 1.
FIG. 3 is a structural diagram of the section B-B' of the sensor electrode structure A in Embodiment 1.
FIG. 4 is a structural diagram of the section C-C' of the sensor electrode structure A in Embodiment 1.
FIG. 5 is a view of the sensor electrode structure B in Embodiment 2.
FIG. 6 is a structural diagram of the section A-A' of the sensor electrode structure B in Embodiment 2.
FIG. 7 is a structural diagram of the section B-B' of the sensor electrode structure B in Embodiment 2.
FIG. 8 is a structural diagram of the section C-C' of the sensor electrode structure B in Embodiment 2.
FIG. 9 is a view of the sensor electrode structure C in Embodiment 3.
FIG. 10 is a structural diagram of the section A-A' of the sensor electrode structure C in Embodiment 3.
FIG. 11 is a structural diagram of the section B-B' of the sensor electrode structure C in Embodiment 3.
FIG. 12 is a structural diagram of the section C-C' of the sensor electrode structure C in Embodiment 3.
FIG. 13 is a view of the sensor electrode structure D in Embodiment 4.
FIG. 14 is a structural diagram of the section A-A' of the sensor electrode structure D in Embodiment 4.
FIG. 15 is a structural diagram of the section B-B' of the sensor electrode structure D in Embodiment 4.
FIG. 16 is a structural diagram of the section C-C' of the sensor electrode structure D in Embodiment 4.
FIG. 17 is a view of the sensor electrode structure E in Embodiment 5.
FIG. 18 is a structural diagram of the section A-A' of the sensor electrode structure E in Embodiment 5.
FIG. 19 is a structural diagram of the section B-B' of the sensor electrode structure E in Embodiment 5.
FIG. 20 is a structural diagram of the section C-C' of the sensor electrode structure E in Embodiment 5.
FIG. 21 is a view of the sensor electrode structure F in Embodiment 6.
FIG. 22 is a structural diagram of the section A-A' of the sensor electrode structure F in Embodiment 6.
FIG. 23 is a structural diagram of the section B-B' of the sensor electrode structure F in Embodiment 6.
FIG. 24 is a structural diagram of the section C-C' of the sensor electrode structure F in Embodiment 6.
FIG. 25 is a view of the sensor electrode structure G in Embodiment 7.
FIG. 26 is a structural diagram of the section A-A' of the sensor electrode structure G in Embodiment 7.
FIG. 27 is a structural diagram of the section B-B' of the sensor electrode structure G in Embodiment 7.
FIG. 28 is a structural diagram of the section C-C' of the sensor electrode structure G in Embodiment 7.
FIG. 29 is a view of the sensor electrode structure H in Embodiment 8.
FIG. 30 is a structural diagram of the section A-A' of the sensor electrode structure H in Embodiment 8.
FIG. 31 is a structural diagram of the section B-B' of the sensor electrode structure H in Embodiment 8.
FIG. 32 is a structural diagram of the section C-C' of the sensor electrode structure H in Embodiment 8.
FIG. 33 is a view of the sensor electrode structure I in Embodiment 9.
FIG. 34 is a structural diagram of the section A-A' of the sensor electrode structure I in Embodiment 9.
FIG. 35 is a structural diagram of the section B-B' of the sensor electrode structure I in Embodiment 9.
FIG. 36 is a structural diagram of the section C-C' of the sensor electrode structure I in Embodiment 9.
FIG. 37 is a partial view of the protrusion position of the sensor of the present application.
FIG. 38 is a schematic diagram of the combination of the sensor with a protruding structure and the auxiliary device (see left), as well as a schematic diagram of the combination of the sensor without a protruding structure and the auxiliary device (see right), where the reference number 1000 is the auxiliary device, and 1001 is the sensor.
FIG. 39 is a structural diagram of the sensor surface after implantation of the sensor with the protruding structure into the human body (see left), as well as a structural diagram of the sensor surface after implantation of the sensor without the protruding structure into the human body (see right).
FIG. 40 is a structural diagram of the sensor after implantation of the sensor with the channel structure into the human body (see left), as well as a structural diagram of the sensor after implantation of the sensor without the channel structure (i.e., stacked structure) into the human body (see right).
FIG. 41 is an enlarged partial view of the sensor according to the preferred solution of the present application.
FIG. 42 is a change trend chart of blood glucose monitoring using the first working electrode of the sensor electrode structure implanted into the human body for one day in Comparative Example 1.
FIG. 43 is a change trend chart of blood glucose monitoring using the first working electrode of the sensor electrode structure implanted into the human body for four days in Comparative Example 2.

### Detailed Description of the Preferred Embodiments

### <analyte sensor>

As mentioned earlier, the present application provides an analyte sensor, characterized in that, the analyte sensor comprises a substrate 100, a first working electrode, a second working electrode, a reference electrode, a counter electrode, a first sensing layer 115 and a second sensing layer 122; the first sensing layer 115 is provided on the first working electrode, and the second sensing layer 122 is provided on the second working electrode;
any two of the first working electrode, the second working electrode, the reference electrode, and the counter electrode are located on a first surface of the substrate, and the remaining two electrodes of the first working electrode, the second working electrode, the reference electrode, and the counter electrode are located on a second surface of the substrate opposite to the first surface;
the two electrodes located on the first surface of the substrate are spatially separated by a channel, and/or, the two electrodes located on the second surface of the substrate opposite to the first surface are spatially separated by a channel;
the width of the channel is 1µm-280µm; the length of the channel is 30µm-10,000µm.

According to the embodiments of the present application, any two of the first working electrode, the second working electrode, the reference electrode, and the counter electrode are located on a first surface of the substrate, and the remaining two electrodes of the first working electrode, the second working electrode, the reference electrode, and the counter electrode are located on a second surface of the substrate opposite to the first surface; the two electrodes located on the first surface of the substrate are spatially separated by a channel, and/or, the two electrodes located on the second surface of the substrate opposite to the first surface are spatially separated by a channel; such a structure results in two different functional areas/electrode areas on the first surface of the sensor, and/or there are two different functional areas/electrode areas on the second surface of the sensor opposite to the first surface, which are spatially separated by a channel and do not affect each other; this enables the functions of two different functional areas/electrode areas to be achieved on the same side surface of the sensor, effectively reducing the risk of sensor failure caused by contact between the two different functional areas/electrode areas; moreover, such a structure can ensure that the functional area/electrode area of the sensor has sufficient area, thereby enabling the sensor to have good stability during long-term monitoring. It can effectively avoid the problems of short circuits and open circuits caused by the limited implantation width of the sensor itself and the narrow electrode cables when the first working electrode, the second working electrode, the reference electrode, and the counter electrode are provided on the same surface. In addition, the structure of two different functional areas/electrode areas located on the same side surface of the sensor makes the preparation process of the sensor relatively simple, especially compared to sensors designed according to a stacked structure (i.e. any two electrodes are in a vertical relationship in space, and then separated by a dielectric layer in the middle; this stacked structure increases the manufacturing process flow and difficulty of the sensor, and the thickness will be thicker, affecting the wearing experience); the structure of two different functional areas/electrode areas on the same side surface of the sensor also ensures that the obtained sensor has good flexibility, smaller size, lower cost, and better wearing comfort; moreover, due to the provision of the reference electrode, the analyte sensor can achieve long-term monitoring of the analyte after implantation into the body.

According to the embodiments of the present application, the first working electrode, the second working electrode, the reference electrode, and the counter electrode are provided at both sides of the substrate respectively, forming a structure in which two electrodes are provided on the first surface of the substrate, and two electrodes are provided on the second surface of the substrate opposite to the first surface; the two electrodes provided on the first surface of the substrate refer to any two of the first working electrode, the second working electrode, the reference electrode, and the counter electrode; the two electrodes provided on the second surface of the substrate opposite to the first surface refer to the remaining two electrodes except for the above two electrodes.

According to the embodiments of the present application, the first working electrode and the reference electrode are provided on the first surface of the substrate, and the counter electrode and the second working electrode are provided on the second surface of the substrate opposite to the first surface; or, the first working electrode and the counter electrode are provided on the first surface of the substrate, and the reference electrode and the second working electrode are provided on the second surface of the substrate opposite to the first surface; or, the counter electrode and the reference electrode are provided on the first surface of the substrate, and the first working electrode and the second working electrode are provided on the second surface of the substrate opposite to the first surface.

According to the embodiments of the present application, the first working electrode and the reference electrode are provided on the first surface of the substrate, and the counter electrode and the second working electrode are provided on the second surface of the substrate opposite to the first surface; the first working electrode and the reference electrode are spatially separated by a channel, and/or, the counter electrode and the second working electrode are spatially separated by a channel; or,
the first working electrode and the counter electrode are provided on the first surface of the substrate, and the reference electrode and the second working electrode are provided on the second surface of the substrate opposite to the first surface; the first working electrode and the counter electrode are spatially separated by a channel, and/or, the reference electrode and the second working electrode are spatially separated by a channel; or,
the counter electrode and the reference electrode are provided on the first surface of the substrate, and the first working electrode and the second working electrode are provided on the second surface of the substrate opposite to the first surface; the counter electrode and the reference electrode are spatially separated by a channel, and/or, the first working electrode and the second working electrode are spatially separated by a channel;

According to the embodiments of the present application, the first working electrode and the reference electrode are provided on the first surface of the substrate, and the counter electrode and the second working electrode are provided on the second surface of the substrate opposite to the first surface; the first working electrode and the reference electrode are spatially separated by a channel, and the counter electrode and the second working electrode are spatially separated by a channel; or, the first working electrode and the reference electrode are spatially separated by a channel, and the counter electrode and the second working electrode are stacked on the second surface of the substrate opposite to the first surface and separated by a dielectric layer; or, the first working electrode and the reference electrode are stacked on the first surface of the substrate and separated by a dielectric layer, while the counter electrode and the second working electrode are spatially separated by a channel.

According to the embodiments of the present application, the first working electrode and the counter electrode are provided on the first surface of the substrate, and the reference electrode and the second working electrode are provided on the second surface of the substrate opposite to the first surface; the first working electrode and the counter electrode are spatially separated by a channel, and the reference electrode and the second working electrode are spatially separated by a channel; or, the first working electrode and the counter electrode are spatially separated by a channel, and the reference electrode and the second working electrode are stacked on the second surface of the substrate opposite to the first surface and separated by a dielectric layer; or, the first working electrode and the counter electrode are stacked on the first surface of the substrate and separated by a dielectric layer, while the reference electrode and the second working electrode are spatially separated by a channel.

According to the embodiments of the present application, the counter electrode and the reference electrode are provided on the first surface of the substrate, and the first working electrode and the second working electrode are provided on the second surface of the substrate opposite to the first surface; the counter electrode and the reference electrode are spatially separated by a channel, and the first working electrode and the second working electrode are spatially separated by a channel; or, the counter electrode and the reference electrode are spatially separated by a channel, and the first working electrode and the second working electrode are stacked on the second surface of the substrate opposite to the first surface and separated by a dielectric layer; or, the counter electrode and the reference electrode are stacked on the first surface of the substrate and separated by a dielectric layer, while the first working electrode and the second working electrode are spatially separated by a channel. According to the embodiments of the present application, any two electrodes located at the same side of the substrate and spatially separated by a channel are on the same plane; for example, the electrode layers, electrode cables and electrode contacts of any two electrodes are on the same plane.

According to the embodiments of the present application, the two electrodes located on the same side of the substrate and spatially separated by channel are partially on the same plane; for example, the electrode layers of the two electrodes are not on the same plane, but the electrode cables and electrode contacts of the two electrodes are on the same plane; or, the electrode cables of the two electrodes are not on the same plane, but the electrode layers and electrode contacts of the two electrodes are on the same plane; or, the electrode contacts of the two electrodes are not on the same plane, but the electrode cables and electrode layers of the two electrodes are on the same plane; or, the electrode layers and electrode contacts of the two electrodes are not on the same plane, but the electrode cables of the two electrodes are on the same plane; or, the electrode cables and electrode contacts of the two electrodes are not on the same plane, but the electrode layers of the two electrodes are on the same plane; or, the electrode cables and electrode layers of the two electrodes are not on the same plane, but the electrode contacts of the two electrodes are on the same plane.

According to the embodiments of the present application, any two electrodes located at the same side of the substrate and stacked on each other are on the same plane and separated by a dielectric layer; for example, the electrode layers, electrode cables and electrode contacts of any two electrodes are on the same plane and separated by a dielectric layer.

According to the embodiments of the present application, any two electrodes located at the same side of the substrate and spatially separated by a channel are not stacked on each other.

### <analyte sensor>

The present application provides an analyte sensor, characterized in that, the analyte sensor comprises a substrate, a first working electrode, a second working electrode, a reference electrode, a counter electrode, a first sensing layer 115 and a second sensing layer 122; the first sensing layer 115 is provided on the first working electrode, and the second sensing layer 122 is provided on the second working electrode;
any three of the first working electrode, the second working electrode, the reference electrode, and the counter electrode are located on a first surface of the substrate, and the remaining one electrode of the first working electrode, the second working electrode, the reference electrode, and the counter electrode is located on a second surface of the substrate opposite to the first surface;
one of the three electrodes located on the first surface of the substrate is provided on the surface of the substrate, and the remaining two electrodes are stacked on the surface of the one electrode and separated from the one electrode by a dielectric layer; the remaining two electrodes are spatially separated by a channel;
the width of the channel is 1µm-280µm; the length of the channel is 30µm-10,000µm.

According to the embodiments of the present application, any three of the first working electrode, the second working electrode, the reference electrode, and the counter electrode are located on a first surface of the substrate, and the remaining one electrode of the first working electrode, the second working electrode, the reference electrode, and the counter electrode is located on a second surface of the substrate opposite to the first surface; one of the three electrodes located on the first surface of the substrate is provided on the surface of the substrate, and the remaining two electrodes are stacked on the surface of the one electrode and separated from the one electrode by a dielectric layer; the remaining two electrodes are spatially separated by a channel; such a structure results in three different functional areas/electrode areas on the first surface of the sensor and one functional area/electrode area on a second surface of the sensor opposite to the first surface, and the different functional areas/electrode areas are spatially separated by a channel and do not affect each other; this enables the functions of two different functional areas/electrode areas to be achieved on the same side surface of the sensor, effectively reducing the risk of sensor failure caused by contact between the two different functional areas/electrode areas; moreover, such a structure can ensure that the functional area/electrode area of the sensor has sufficient area, thereby enabling the sensor to have good stability during long-term monitoring. It can effectively avoid the problems of short circuits and open circuits caused by the limited implantation width of the sensor itself and the narrow electrode cables when the first working electrode, the second working electrode, the reference electrode, and the counter electrode are provided on the same surface. In addition, the structure of different functional areas/electrode areas located on the same side surface of the sensor makes the preparation process of the sensor relatively simple, especially compared to sensors designed according to a stacked structure (i.e. any three electrodes are in a vertical relationship in space, and then separated by a dielectric layer; this stacked structure increases the manufacturing process flow and difficulty of the sensor, and the thickness will be thicker, affecting the wearing experience); the structure of different functional areas/electrode areas on the same side surface of the sensor also ensures that the obtained sensor has good flexibility, smaller size, lower cost, and better wearing comfort; moreover, due to the provision of the reference electrode, the analyte sensor can achieve long-term monitoring of the analyte after implantation into the body.

According to the embodiments of the present application, the first working electrode, the second working electrode, the reference electrode, and the counter electrode are provided at both sides of the substrate respectively, forming a structure in which three electrodes are provided on the first surface of the substrate, and one electrode is provided on the second surface of the substrate opposite to the first surface; the three electrodes provided on the first surface of the substrate refer to any three of the first working electrode, the second working electrode, the reference electrode, and the counter electrode; the one electrode provided on the second surface of the substrate opposite to the first surface refer to the remaining one electrode except for the above three electrodes.

According to the embodiments of the present application, the first working electrode, the second working electrode, and the reference electrode are provided on the first surface of the substrate, and the counter electrode is provided on the second surface of the substrate opposite to the first surface; or, the first working electrode, the second working electrode, and the counter electrode are provided on the first surface of the substrate, and the reference electrode is provided on the second surface of the substrate opposite to the first surface; or, the first working electrode, the counter electrode, and the reference electrode are provided on the first surface of the substrate, and the second working electrode is provided on the second surface of the substrate opposite to the first surface; or, the second working electrode, the counter electrode, and the reference electrode are provided on the first surface of the substrate, and the first working electrode is provided on the second surface of the substrate opposite to the first surface.

According to the embodiments of the present application, the first working electrode, the second working electrode, and the reference electrode are provided on the first surface of the substrate, and the counter electrode is provided on the second surface of the substrate opposite to the first surface; the first working electrode is provided on the surface of the substrate, the second working electrode and the reference electrode are stacked on the surface of the first working electrode respectively and separated by a dielectric layer, and the second working electrode and the reference electrode are spatially separated by a channel. Furthermore, preferably the first working electrode is closer to the front end 101 of the substrate than the second working electrode and the reference electrode; or, the second working electrode is provided on the surface of the substrate, and the first working electrode and the reference electrode are stacked on the surface of the second working electrode respectively and separated by a dielectric layer. The first working electrode and the reference electrode are spatially separated by a channel. Furthermore, preferably the second working electrode is closer to the front end 101 of the substrate than the first working electrode and the reference electrode; or, the reference electrode is provided on the surface of the substrate, and the first working electrode and the second working electrode are stacked on the surface of the reference electrode respectively and separated by a dielectric layer. The first working electrode and the second working electrode are spatially separated by a channel, and furthermore, preferably the reference electrode is closer to the front end 101 of the substrate than the first working electrode and the second working electrode. According to the embodiments of the present application, the first working electrode, the second working electrode, and the counter electrode are provided on the first surface of the substrate, and the reference electrode is provided on the second surface of the substrate opposite to the first surface; the first working electrode is provided on the surface of the substrate, the second working electrode and the counter electrode are stacked on the surface of the first working electrode respectively and separated by a dielectric layer, and the second working electrode and the counter electrode are spatially separated by a channel. Furthermore, preferably the first working electrode is closer to the front end 101 of the substrate than the second working electrode and the counter electrode; or, the second working electrode is provided on the surface of the substrate, and the first working electrode and the counter electrode are stacked on the surface of the second working electrode respectively and separated by a dielectric layer. The first working electrode and the counter electrode are spatially separated by a channel. Furthermore, preferably the second working electrode is closer to the front end 101 of the substrate than the first working electrode and the counter electrode; or, the counter electrode is provided on the surface of the substrate, and the first working electrode and the second working electrode are stacked on the surface of the counter electrode respectively and separated by a dielectric layer. The first working electrode and the second working electrode are spatially separated by a channel, and furthermore, preferably the counter electrode is closer to the front end 101 of the substrate than the first working electrode and the second working electrode.

According to the embodiments of the present application, the first working electrode, the reference electrode, and the counter electrode are provided on the first surface of the substrate, and the second working electrode is provided on the second surface of the substrate opposite to the first surface; the first working electrode is provided on the surface of the substrate, the reference electrode and the counter electrode are stacked on the surface of the first working electrode respectively and separated by a dielectric layer, and the reference electrode and the counter electrode are spatially separated by a channel. Furthermore, preferably the first working electrode is closer to the front end 101 of the substrate than the counter electrode and the reference electrode; or, the reference electrode is provided on the surface of the substrate, and the first working electrode and the counter electrode are stacked on the surface of the reference electrode respectively and separated by a dielectric layer. The first working electrode and the counter electrode are spatially separated by a channel. Furthermore, preferably the reference electrode is closer to the front end 101 of the substrate than the first working electrode and the counter electrode; or, the counter electrode is provided on the surface of the substrate, and the first working electrode and the reference electrode are stacked on the surface of the counter electrode respectively and separated by a dielectric layer. The first working electrode and the reference electrode are spatially separated by a channel, and furthermore, preferably the counter electrode is closer to the front end 101 of the substrate than the first working electrode and the reference working electrode.

According to the embodiments of the present application, the second working electrode, the reference electrode, and the counter electrode are provided on the first surface of the substrate, and the first working electrode is provided on the second surface of the substrate opposite to the first surface; the second working electrode is provided on the surface of the substrate, the reference electrode and the counter electrode are stacked on the surface of the second working electrode respectively and separated by a dielectric layer, and the reference electrode and the counter electrode are spatially separated by a channel. Furthermore, preferably the second working electrode is closer to the front end 101 of the substrate than the reference electrode and the counter electrode; or, the reference electrode is provided on the surface of the substrate, and the second working electrode and the counter electrode are stacked on the surface of the reference electrode respectively and separated by a dielectric layer. The second working electrode and the counter electrode are spatially separated by a channel. Furthermore, preferably the reference electrode is closer to the front end 101 of the substrate than the second working electrode and the counter electrode; or, the counter electrode is provided on the surface of the substrate, and the second working electrode and the reference electrode are stacked on the surface of the counter electrode respectively and separated by a dielectric layer. The second working electrode and the reference electrode are spatially separated by a channel, and furthermore, preferably the counter electrode is closer to the front end 101 of the substrate than the second working electrode and the reference working electrode.

According to the embodiments of the present application, any two of three electrodes located at the same side of the substrate and spatially separated by a channel may be completely on the same plane; for example, the electrode layers, electrode cables and electrode contacts of any two electrodes are on the same plane.

According to the embodiments of the present application, any two of three electrodes located on the same side of the substrate and spatially separated by channel may be partially on the same plane; for example, the electrode layers of any two electrodes are not on the same plane, but the electrode cables and electrode contacts of any two electrodes are on the same plane; or, the electrode cables of any two electrodes are not on the same plane, but the electrode layers and electrode contacts of any two electrodes are on the same plane; or, the electrode contacts of any two electrodes are not on the same plane, but the electrode cables and electrode layers of the any two electrodes are on the same plane; or, the electrode layers and electrode contacts of any two electrodes are not on the same plane, but the electrode cables of the any two electrodes are on the same plane; or, the electrode cables and electrode contacts of any two electrodes are not on the same plane, but the electrode layers of any two electrodes are on the same plane; or, the electrode cables and electrode layers of any two electrodes are not on the same plane, but the electrode contacts of any two electrodes are on the same plane.

According to the embodiments of the present application, any two electrodes located at the same side of the substrate and spatially separated by a channel are not stacked on each other.

### <structure and composition of the substrate>

According to the embodiments of the present application, the substrate 100 comprises a front end 101, a middle end 102, and a distal end 103, which are sequentially connected.

According to the embodiments of the present application, the analyte sensor 100 has an L-shape, which includes a first side and a second side, and an angle of 45°-135° is formed at the connection between the first side and the second side, preferably 88°-98°, such as 90°, 92°, 93°, 94°, 95°, 96°, 97° or 98°.

According to the embodiments of the present application, the front end 101 is located at the first side and at the non-connected end of the first side, the distal end 103 is located at the second side and at the non-connected end of the second side, and the middle end 102 is located at the first side and second side and at the connected end of the first side and second side. The connected end refers to the end close to the connection between the first side and the second side, while the non-connected end refers to the end far away from the connection between the first side and the second side.

According to the embodiments of the present application, the material forming the substrate comprises at least one of plastic (such as polyethylene terephthalate PET, polyimide PI, polyvinyl chloride PVC, or polyethylene PE), polydimethylsiloxane PDMS, ceramics, glass, paper, and textiles.

According to the embodiments of the present application, the length of the second side of the L-shaped substrate is 1mm-40mm, the length of the first side of the L-shaped substrate is 0.05mm-20mm, and the thickness of the substrate is 20µm-5000µm; preferably, the length of the second side of the L-shaped substrate is 2mm-30mm, the length of the first side of the L-shaped substrate is 0.10mm-10mm, and the thickness of the substrate is 50µm-2000µ m; for example, the length of the second side of the L-shaped substrate is 3mm, 4mm, 5mm, 8mm, 10mm, 12mm, 15mm, 18mm, 20mm, 25mm, 28mm, or 30mm; the length of the first side of the L-shaped substrate is 0.10mm, 0.5mm, 1mm, 2mm, 3mm, 4mm, 5mm, 6mm, 8mm, or 10mm.

### <structure and composition of the working electrode>

According to the embodiments of the present application, the first working electrode comprises a first working electrode layer 107, a first working electrode cable 108, and a first working electrode contact 109, and the first working electrode layer 107 is connected to the first working electrode contact 109 through the first working electrode cable 108.

For example, the first working electrode layer 107 is located at the front end 101 of the substrate, the first working electrode cable 108 is located at the middle end 102 of the substrate, and the first working electrode contact 109 is located at the distal end 103 of the substrate; or, the first working electrode layer 107 and the first working electrode cable 108 are located at the middle end 102 of the substrate, and the first working electrode contact 109 is located at the distal end 103 of the substrate.

According to the embodiments of the present application, a first sensing layer 115 is provided on the first working electrode layer 107, and the first working electrode mainly functions to react with a first analyte.

According to the embodiments of the present application, the second working electrode comprises a second working electrode layer 119, a second working electrode cable 120, and a second working electrode contact 121, and the second working electrode layer 119 is connected to the second working electrode contact 121 through the second working electrode cable 120.

For example, the second working electrode layer 119 is located at the front end 101 of the substrate, the second working electrode cable 120 is located at the middle end 102 of the substrate, and the second working electrode contact 121 is located at the distal end 103 of the substrate; or, the second working electrode layer 119 and the second working electrode cable 120 are located at the middle end 102 of the substrate, and the second working electrode contact 121 is located at the distal end 103 of the substrate.

According to the embodiments of the present application, a second sensing layer 122 is provided on the second working electrode layer 119, and the second working electrode mainly functions to react with a second analyte.

According to the embodiments of the present application, the material forming the working electrode comprises one or more of carbon, gold, silver, copper, and their composite materials; or, the material forming the working electrode comprises one or more of precious metals, transition metals, transition metal oxides, and transition metal hydroxides with catalytic/signal conversion properties. For example, the precious metals include gold and its composite materials, platinum and its composite materials; the transition metals include copper and its composite materials, nickel and its composite materials, cobalt and its composite materials, zinc and its composite materials, manganese and its composite materials, etc.

According to the embodiments of the present application, the working electrode is modified onto the substrate through process methods including but not limited to printing, inkjet printing, electrohydrodynamic (EHD) printing, dispensing, spraying, chemical vapor deposition (CVD), physical vapor deposition (PVD), magnetron sputtering, molecular beam epitaxy, electrochemical deposition, electroplating, and evaporation deposition. According to the embodiments of the present application, the material forming the first working electrode and the material forming the second working electrode are the same or different.

According to the embodiments of the present application, the method for preparing the first working electrode and the method for preparing the second working electrode are the same or different.

According to the embodiments of the present application, the thickness of the first working electrode layer is 1µm-20µm, preferably 5µm-15µm, such as 2µm, 3µm, 4µm, 5µm, 6µm, 7µm, 8µm, 9µm, 10µm, 11µm, 12µm, 15µm or 18µm.

According to the embodiments of the present application, the thickness of the second working electrode layer is 1µm-20µm, preferably 5µm-15µm, such as 2µm, 3µm, 4µm, 5µm, 6µm, 7µm, 8µm, 9µm, 10µm, 11µm, 12µm, 15µm or 18µm.

According to the embodiments of the present application, the thickness of the first working electrode layer and the thickness of the second working electrode layer are the same or different.

### <structure and composition of the reference electrode>

According to the embodiments of the present application, the reference electrode comprises a reference electrode layer 113, a reference electrode cable 110, and a reference electrode contact 112. The reference electrode layer 113 is provided on the reference electrode cable 110, and the reference electrode cable 110 is connected to the reference electrode contact 112.

For example, the reference electrode layer 113 is located at the front end 101 of the substrate, the reference electrode cable 110 is located at the front end 101 and middle end 102 of the substrate, and the reference electrode contact 112 is located at the distal end 103 of the substrate; or, the reference electrode layer 113 and the reference electrode cable 110 are located at the middle end 102 of the substrate, and the reference electrode contact 112 is located at the distal end 103 of the substrate.

According to the embodiments of the present application, the reference electrode is configured to form a voltage circuit with the first working electrode, providing a guarantee for converting a first analyte signal into an electrochemical signal, and further configured to form a voltage circuit with the second working electrode, providing a guarantee for converting a second analyte signal into an electrochemical signal. The reference electrode plays a role in stabilizing the potential, and thus the analyte sensor has the function of continuous and stable monitoring.

According to the embodiments of the present application, the material forming the reference electrode comprises Ag and AgCl, where the mass ratio of Ag to AgCl is 1:10-10:1, preferably 2:8-8:2, such as 3:7, 4:6, 5:5, 6:4, 7:3.

According to the embodiments of the present application, the reference electrode is modified onto the substrate through process methods including but not limited to printing, inkjet printing, electrohydrodynamic (EHD) printing, dispensing, spraying, chemical vapor deposition (CVD), physical vapor deposition (PVD), magnetron sputtering, molecular beam epitaxy, electrochemical deposition, electroplating, and evaporation deposition. According to the embodiments of the present application, the thickness of the reference electrode layer is 0.1µm-500µm, preferably 1µm-200µm, such as 5µm, 10µm, 15µm, 20µm, 50µm, 80µm, 90µm, 100µm, 120µm, 150µm, 180µm or 200µm.

### <structure and composition of the counter electrode>

According to the embodiments of the present application, the counter electrode comprises a counter electrode layer 104, a counter electrode cable 105, and a counter electrode contact 106. The counter electrode layer 104 is connected to the counter electrode contact 106 through the counter electrode cable 105.

For example, the counter electrode layer 104 is located at the front end 101 of the substrate, the counter electrode cable 105 is located at the middle end 102 of the substrate, and the counter electrode contact 106 is located at the distal end 103 of the substrate; or, the counter electrode layer 104 and the counter electrode cable 105 are located at the middle end 102 of the substrate, and the counter electrode contact 106 is located at the distal end 103 of the substrate.

According to the embodiments of the present application, the counter electrode is configured to form a current closed circuit with the first working electrode, providing a guarantee for converting a first analyte signal into an electrochemical signal, and further configured to form a current closed circuit with the second working electrode, providing a guarantee for converting a second analyte signal into an electrochemical signal.

According to the embodiments of the present application, the material forming the counter electrode comprises one or more of carbon, gold, silver, copper, and their composite materials.

According to the embodiments of the present application, the counter electrode is modified onto the substrate through process methods including but not limited to printing, inkjet printing, electrohydrodynamic (EHD) printing, dispensing, spraying, chemical vapor deposition (CVD), physical vapor deposition (PVD), magnetron sputtering, molecular beam epitaxy, electrochemical deposition, electroplating, and evaporation deposition. According to the embodiments of the present application, the thickness of the counter electrode layer is 1µm-20µm, preferably 5µm-15µm, such as 2µm, 3µm, 4µm, 5µm, 6µm, 7µm, 8µm, 9µm, 10µm, 11µm, 12µm, 15µm or 18µm.

### <sensing layer>

According to the embodiments of the present application, the first sensing layer 115 and the second sensing layer 122 are the same or different. In order to achieve testing of different analytes, the first sensing layer 115 and the second sensing layer 122 are preferably different.

According to the embodiments of the present application, the first sensing layer 115 is provided on the first working electrode layer 107 of the first working electrode; the second sensing layer 122 is provided on the second working electrode layer 119 of the second working electrode.

According to the embodiments of the present application, the material forming the sensing layer comprises one or more of carbon, gold, silver, copper, and their composite materials; or, the material forming the working electrode comprises one or more of biological enzymes, precious metals, transition metals, transition metal oxides, and transition metal hydroxides. For example, the biological enzymes include glucose oxidase, glucose dehydrogenase, uricase, lactate enzyme, β-hydroxybutyrate dehydrogenase, etc.; the precious metals include gold and its composite materials, platinum and its composite materials; the precious metals include gold and its composite materials, platinum and its composite materials; the transition metals include copper and its composite materials, nickel and its composite materials, cobalt and its composite materials, osmium and its composite materials, zinc and its composite materials, manganese and its composite materials, iron and its composite materials, etc.

According to the embodiments of the present application, the first sensing layer 115 is formed on the first working electrode layer of the first working electrode through process methods including but not limited to adsorption, printing, inkjet printing, electrohydrodynamic (EHD) printing, dispensing, spraying, chemical vapor deposition (CVD), physical vapor deposition (PVD), magnetron sputtering, molecular beam epitaxy, electrochemical deposition, and evaporation deposition.

According to the embodiments of the present application, the second sensing layer 122 is formed on the second working electrode layer of the second working electrode through process methods including but not limited to adsorption, printing, inkjet printing, electrohydrodynamic (EHD) printing, dispensing, spraying, chemical vapor deposition (CVD), physical vapor deposition (PVD), magnetron sputtering, molecular beam epitaxy, electrochemical deposition, and evaporation deposition.

According to the embodiments of the present application, the thickness of the first sensing layer 115 and the thickness of the second sensing layer 122 are the same or different.

According to the embodiments of the present application, the thickness of the first sensing layer 115 is 1µm-20µm, preferably 1µm-10µm, such as 2µm, 3µm, 4µm, 5µm, 6µm, 7µm, 8µm, 9µm, 10µm, 11µm, 12µm,15µm or 18µm. According to the embodiments of the present application, the thickness of the second sensing layer 122 is 1µm-20µm, preferably 1µm-10µm, such as 2µm, 3µm, 4µm, 5µm, 6µm, 7µm, 8µm, 9µm, 10µm, 11µm, 12µm, 15µm or 18µm.

According to the embodiments of the present application, the first sensing layer and the second sensing layer serve to continuously monitor multiple analytes.

According to the embodiments of the present application, the analyte/physiological indicator that can be monitored by the first sensing layer and the second sensing layer includes one or more of blood glucose, uric acid, lactic acid, ketones, cholesterol, triglycerides, heart rate, blood pressure, Na⁺, Ka⁺, Ga²⁺, Fe²⁺ and Mg²⁺, etc.

For example, the analyte/physiological indicator that can be monitored by the first sensing layer is blood glucose; the analyte/physiological indicator that can be monitored by the second sensing layer includes one of uric acid, lactic acid, ketones, cholesterol, triglycerides, heart rate, blood pressure, Na⁺, Ka⁺, Ga²⁺, Fe²⁺ and Mg²⁺, etc.

### <provision of the electrode>

In terms of the present application, the two electrodes spatially separated by a channel refer to that the electrode layer of one electrode and the electrode cable of the other electrode are spatially separated by a channel;

According to the embodiments of the present application, the two electrodes spatially separated by a channel are, for example, a first working electrode and a reference electrode, and the first working electrode layer 107 of the first working electrode is located at the front end 101 of the substrate; the reference electrode layer 113 of the reference electrode is located at the middle end 102 of the substrate, and the reference electrode and the first working electrode are spatially separated by a channel; or, the first working electrode layer 107 of the first working electrode is located at the middle end 102 of the substrate, the reference electrode layer 113 of the reference electrode is located at the front end 101 of the substrate, and the reference electrode and the first working electrode are spatially separated by a channel.

According to the embodiments of the present application, the two electrodes spatially separated by a channel are, for example, a second working electrode and a reference electrode, and the second working electrode layer 119 of the second working electrode is located at the front end 101 of the substrate; the reference electrode layer 113 of the reference electrode is located at the middle end 102 of the substrate, and the reference electrode and the second working electrode are spatially separated by a channel; or, the second working electrode layer 119 of the second working electrode is located at the middle end 102 of the substrate, the reference electrode layer 113 of the reference electrode is located at the front end 101 of the substrate, and the reference electrode and the second working electrode are spatially separated by a channel.

According to the embodiments of the present application, the two electrodes spatially separated by a channel are, for example, a first working electrode and a counter electrode, and the first working electrode layer 107 of the first working electrode is located at the front end 101 of the substrate; the counter electrode layer 104 of the counter electrode is located at the middle end 102 of the substrate, and the counter electrode and the first working electrode are spatially separated by a channel; or, the first working electrode layer 107 of the first working electrode is located at the middle end 102 of the substrate, the counter electrode layer 104 of the counter electrode is located at the front end 101 of the substrate, and the counter electrode and the first working electrode are spatially separated by a channel.

According to the embodiments of the present application, the two electrodes spatially separated by a channel are, for example, a second working electrode and a counter electrode, and the second working electrode layer 119 of the second working electrode is located at the front end 101 of the substrate; the counter electrode layer 104 of the counter electrode is located at the middle end 102 of the substrate, and the counter electrode and the second working electrode are spatially separated by a channel; or, the second working electrode layer 119 of the second working electrode is located at the middle end 102 of the substrate, the counter electrode layer 104 of the counter electrode is located at the front end 101 of the substrate, and the counter electrode and the second working electrode are spatially separated by a channel.

According to the embodiments of the present application, the two electrodes spatially separated by a channel are, for example, a counter electrode and a reference electrode, and the reference electrode layer 113 of the reference electrode is located at the front end 101 of the substrate; the counter electrode layer 104 of the counter electrode is located at the middle end 102 of the substrate, and the reference electrode and the counter electrode are spatially separated by a channel; or, the reference electrode layer 113 of the reference electrode is located at the middle end 102 of the substrate, the counter electrode layer 104 of the counter electrode is located at the front end 101 of the substrate, and the reference electrode and the counter electrode are spatially separated by a channel.

According to the embodiments of the present application, the two electrodes spatially separated by a channel are, for example, a first working electrode and a second working electrode, and the first working electrode layer 107 of the first working electrode is located at the front end 101 of the substrate; the second working electrode layer 120 of the second working electrode is located at the middle end 102 of the substrate, and the first working electrode and the second working electrode are spatially separated by a channel; or, the first working electrode layer 107 of the first working electrode is located at the middle end 102 of the substrate, the second working electrode layer 120 of the second working electrode is located at the front end 101 of the substrate, and the first working electrode and the second working electrode are spatially separated by a channel.

According to the embodiments of the present application, when the first working electrode layer 107 of the first working electrode is located at the front end 101 of the substrate, and the reference electrode layer 113 of the reference electrode is located at the middle end 102 of the substrate, the reference electrode layer 113 of the reference electrode and the first working electrode cable 108 of the first working electrode are spatially separated by a channel.

According to the embodiments of the present application, when the first working electrode layer 107 of the first working electrode is located at the middle end 102 of the substrate, and the reference electrode layer 113 of the reference electrode is located at the front end 101 of the substrate, the reference electrode cable 110 of the reference electrode and the first working electrode layer 107 of the first working electrode are spatially separated by a channel.

According to the embodiments of the present application, when the first working electrode layer 107 of the first working electrode is located at the front end 101 of the substrate, and the counter electrode layer 104 of the counter electrode is located at the middle end 102 of the substrate, the counter electrode layer 104 of the counter electrode and the first working electrode cable 108 of the first working electrode are spatially separated by a channel.

According to the embodiments of the present application, when the first working electrode layer 107 of the first working electrode is located at the middle end 102 of the substrate, and the counter electrode layer 104 of the counter electrode is located at the front end 101 of the substrate, the counter electrode cable 105 of the counter electrode and the first working electrode layer 107 of the first working electrode are spatially separated by a channel.

According to the embodiments of the present application, when the second working electrode layer 119 of the second working electrode is located at the front end 101 of the substrate, and the reference electrode layer 113 of the reference electrode is located at the middle end 102 of the substrate, the reference electrode layer 113 of the reference electrode and the second working electrode cable 120 of the second working electrode are spatially separated by a channel.

According to the embodiments of the present application, when the second working electrode layer 119 of the first working electrode is located at the middle end 102 of the substrate, and the reference electrode layer 113 of the reference electrode is located at the front end 101 of the substrate, the reference electrode cable 110 of the reference electrode and the second working electrode layer 119 of the second working electrode are spatially separated by a channel.

According to the embodiments of the present application, when the second working electrode layer 119 of the second working electrode is located at the front end 101 of the substrate, and the counter electrode layer 104 of the counter electrode is located at the middle end 102 of the substrate, the counter electrode layer 104 of the counter electrode and the second working electrode cable 120 of the second working electrode are spatially separated by a channel.

According to the embodiments of the present application, when the second working electrode layer 119 of the second working electrode is located at the middle end 102 of the substrate, and the counter electrode layer 104 of the counter electrode is located at the front end 101 of the substrate, the counter electrode cable 105 of the counter electrode and the second working electrode layer 119 of the second working electrode are spatially separated by a channel.

According to the embodiments of the present application, when the reference electrode layer 113 of the reference electrode is located at the front end 101 of the substrate, and the counter electrode layer 104 of the counter electrode is located at the middle end 102 of the substrate, the counter electrode layer 104 of the counter electrode and the reference electrode cable 110 of the reference electrode are spatially separated by a channel.

According to the embodiments of the present application, when the reference electrode layer 113 of the reference electrode is located at the middle end 102 of the substrate, and the counter electrode layer 104 of the counter electrode is located at the front end 101 of the substrate, the counter electrode cable 105 of the counter electrode and the reference electrode layer 113 of the reference electrode are spatially separated by a channel.

According to the embodiments of the present application, when the first working electrode layer 107 of the first working electrode is located at the front end 101 of the substrate, and the second working electrode layer 119 of the second working electrode is located at the middle end 102 of the substrate, the second working electrode layer 119 of the second working electrode and the first working electrode cable 108 of the first working electrode are spatially separated by a channel.

According to the embodiments of the present application, when the first working electrode layer 107 of the first working electrode is located at the middle end 102 of the substrate, and the second working electrode layer 119 of the second working electrode is located at the front end 101 of the substrate, the second working electrode cable 120 of the second working electrode and the first working electrode layer 107 of the first working electrode are spatially separated by a channel.

According to the embodiments of the present application, when the two electrodes spatially separated by a channel are a first working electrode and a reference electrode, the first working electrode and the reference electrode are on the same plane. Specifically, the first working electrode layer 107 of the first working electrode, the first working electrode cable 108 of the first working electrode, the first working electrode contact 109 of the first working electrode, the reference electrode layer 113 of the reference electrode, the reference electrode cable 110 of the reference electrode, and the reference electrode contact 112 of the reference electrode are all on the same plane; or, when the two electrodes spatially separated by a channel are a first working electrode and a reference electrode, the first working electrode and the reference electrode are partially on the same plane. Specifically, at least one component of the electrode layers, electrode cables and electrode contacts of the first working electrode and the reference electrode are on the same plane, while at least one component of the electrode layers, electrode cables and electrode contacts of the first working electrode and the reference electrode are not on the same plane.

According to the embodiments of the present application, when the two electrodes spatially separated by a channel are a second working electrode and a reference electrode, the second working electrode and the reference electrode are on the same plane. Specifically, the second working electrode layer 119 of the second working electrode, the second working electrode cable 120 of the second working electrode, the second working electrode contact 121 of the second working electrode, the reference electrode layer 113 of the reference electrode, the reference electrode cable 110 of the reference electrode, and the reference electrode contact 112 of the reference electrode are all on the same plane; or, when the two electrodes spatially separated by a channel are a second working electrode and a reference electrode, the second working electrode and the reference electrode are partially on the same plane. Specifically, at least one component of the electrode layers, electrode cables, and electrode contacts of the second working electrode and the reference electrode are on the same plane, while at least one component of the electrode layers, electrode cables, and electrode contacts of the second working electrode and the reference electrode are not on the same plane.

According to the embodiments of the present application, when the two electrodes spatially separated by a channel are a first working electrode and a counter electrode, the first working electrode and the counter electrode are on the same plane. Specifically, the first working electrode layer 107 of the first working electrode, the first working electrode cable 108 of the first working electrode, the first working electrode contact 109 of the first working electrode, the counter electrode layer 104 of the counter electrode, the counter electrode cable 105 of the counter electrode, and the counter electrode contact 106 of the counter electrode are all on the same plane; or, when the two electrodes spatially separated by a channel are a first working electrode and a counter electrode, the first working electrode and the reference electrode are partially on the same plane. Specifically, at least one component of the electrode layers, electrode cables, and electrode contacts of the second working electrode and the counter electrode are on the same plane, while at least one component of the electrode layers, electrode cables, and electrode contacts of the second working electrode and the counter electrode are not on the same plane. According to the embodiments of the present application, when the two electrodes spatially separated by a channel are a second working electrode and a counter electrode, the second working electrode and the counter electrode are on the same plane. Specifically, the second working electrode layer 119 of the second working electrode, the second working electrode cable 120 of the second working electrode, the second working electrode contact 121 of the second working electrode, the counter electrode layer 104 of the counter electrode, the counter electrode cable 105 of the counter electrode, and the counter electrode contact 106 of the counter electrode are all on the same plane; or, when the two electrodes spatially separated by a channel are a second working electrode and a counter electrode, the second working electrode and the reference electrode are partially on the same plane. Specifically, at least one component of the electrode layers, electrode cables, and electrode contacts of the second working electrode and the counter electrode are on the same plane, while at least one component of the electrode layers, electrode cables, and electrode contacts of the second working electrode and the counter electrode are not on the same plane.

According to the embodiments of the present application, when the two electrodes spatially separated by a channel are a counter electrode and a reference electrode, the counter electrode and the reference electrode are on the same plane. Specifically, the counter electrode layer 104 of the counter electrode, the counter electrode cable 105 of the counter electrode, the counter electrode contact 106 of the counter electrode, the reference electrode layer 113 of the reference electrode, the reference electrode cable 110 of the reference electrode, and the reference electrode contact 112 of the reference electrode are all on the same plane; or, when the two electrodes spatially separated by a channel are a counter electrode and a reference electrode, the counter electrode and the reference electrode are partially on the same plane. Specifically, at least one component of the electrode layers, electrode cables, and electrode contacts of the counter electrode and reference electrode are on the same plane; while at least one component of the electrode layers, electrode cables, and electrode contacts of the counter electrode and reference electrode are not on the same plane.

According to the embodiments of the present application, when the two electrodes spatially separated by a channel are a first working electrode and a second working electrode, the first working electrode and the second working electrode are on the same plane. Specifically, the first working electrode layer 107 of the first working electrode, the first working electrode cable 108 of the first working electrode, the first working electrode contact 109 of the first working electrode, the second working electrode layer 119 of the second working electrode, the second working electrode cable 120 of the reference electrode, and the second working electrode contact 121 of the reference electrode are all on the same plane; or, when the two electrodes spatially separated by a channel are a first working electrode and a second working electrode, the first working electrode and the second working electrode are partially on the same plane. Specifically, at least one component of the electrode layers, electrode cables, and electrode contacts of the first working electrode and the second working electrode are on the same plane, while at least one component of the electrode layers, electrode cables, and electrode contacts of the first working electrode and the second working electrode are not on the same plane.

### <structure of the channel>

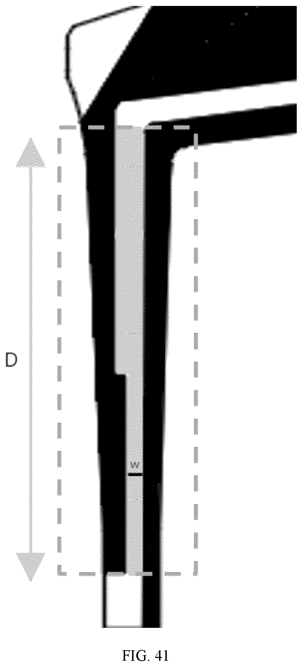

According to the embodiments of the present application, as shown in FIG. 41, the width of the channel 114 refers to the distance between the electrode layer of one electrode and the electrode cable of the other electrode spatially separated by a channel; the line segment "W" in FIG. 41 represents the width of the channel 114; the specific electrode layers and electrode cables need to be determined according to different arrangements of the working electrode, reference electrode, and counter electrode inside the sensor; for example, the width of the channel refers to the distance between the counter electrode cable 105 of the counter electrode and the reference electrode layer 113 of the reference electrode; or the distance between the electrode cable 105 and the first working electrode layer 107 of the working electrode; or the distance between the counter electrode cable 105 of the counter electrode and the second working electrode layer 119 of the second working electrode; or the distance between the reference electrode cable 110 of the reference electrode and the first working electrode layer 107 of the first working electrode; or the distance between the reference electrode cable 110 of the reference electrode and the counter electrode layer 104 of the counter electrode; or the distance between the reference electrode cable 110 of the reference electrode and the second working electrode layer 119 of the second working electrode; or the distance between the first working electrode cable 108 of the first working electrode and the counter electrode layer 104 of the counter electrode; or the distance between the first working electrode cable 108 of the first working electrode and the reference electrode layer 113 of the reference electrode; or the distance between the first working electrode cable 108 of the first working electrode and the second working electrode layer 119 of the second working electrode; or the distance between the second working electrode cable 120 of the second working electrode and the counter electrode layer 104 of the counter electrode; or the distance between the second working electrode cable 120 of the second working electrode and the reference electrode layer 113 of the reference electrode; or the distance between the second working electrode cable 120 of the second working electrode and the first working electrode layer 107 of the first working electrode.

According to the embodiments of the present application, the width of the channel may be constant or gradually changing; if it is constant, the width of the channel is constant within the range of 1µm-280µm; if it is gradually changing, the width of the channel gradually increases or decreases along the direction from the front end 101 of the substrate to the middle end 102 of the substrate, and the width of the channel varies within a width range of 1µm-280µm, for example, within a width range of 50µm-150µm or within a width range of 80µm-200µm. According to the embodiments of the present application, the width of the channel is 1µm-280µm, preferably 50µm-170µm, such as 20µm, 30µm, 40µm, 50µm, 60µm, 70µm, 80µm, 90µm, 100µm, 110µm, 120µm, 130µm, 140µm, 150µm, 160µm, 170µm, 180µm, 190µm, 200µm, 210µm, 220µm, 230µm, 240µm, 250µm, 260µm, 270µm, 280µm, or a range consisting of the two endpoint values mentioned above. Research has found that when the width of the channel is 1µm-280µm, it can effectively separate the electrodes located on the same side surface of the sensor, enabling the functions of multiple functional areas/electrode areas on the same side surface of the sensor and improving the stability of the sensor; when the width of the channel is less than 1µm, due to the narrow width of the channel, there is a risk of contact between the two electrodes, resulting in a short circuit between the electrodes and causing the test data to be too high or too low, and resulting in sensor failure; when the width of the channel is greater than 280µm, due to the wide channel width, the electrode/electrode cable may be too narrow, which may result in insufficient functional area/electrode area, or the electrode may be disconnected due to the narrow electrode/electrode cable, leading to data "jumping" or "jittering" during the sensor testing process, affecting the stability of the test data and causing sensor failure.

According to the embodiments of the present application, as shown in FIG. 41, the length of the channel 114 refers to the distance from the front end of the electrode layer located at the middle end of the substrate to the connection between the first side and the second side of the substrate, that is, the line segment "D" in FIG. 41 represents the length of the channel 114; the specific electrode layer needs to be determined according to different arrangements of the first working electrode, the second working electrode, the reference electrode, and the counter electrode inside the sensor; for example, the length of the channel is the distance from the front end of the first working electrode layer of the first working electrode located at the middle end of the substrate to the connection between the first side and the second side of the substrate; or the length of the channel is the distance from the front end of the second working electrode layer of the second working electrode located at the middle end of the substrate to the connection between the first side and the second side of the substrate; or the distance from the front end of the reference electrode layer of the reference electrode located at the middle end of the substrate to the connection between the first side and the second side of the substrate; or the distance from the front end of the counter electrode layer of the counter electrode located at the middle end of the substrate to the connection between the first side and the second side of the substrate. The front end of the electrode layer refers to the end close to the front end of the substrate.

According to the embodiments of the present application, the length of the channel is 30µm-10,000µm, such as 30µm, 40µm, 50µm, 60µm, 70µm, 80µm, 90µm, 100µm, 110µm, 120µm, 130µm, 140µm, 150µm, 160µm, 170µm, 180µm, 190µm, 200µm, 210µm, 220µm, 230µm, 240µm, 250µm, 260µm, 270µm, 280µm, 300µm, 320µm, 350µm, 380µm, 400µm, 450µm, 500µm, 550µm, 600µm, 700µm, 800µm, 900µm, 1,000µm, 1,100µm, 1,200µm, 1,300µm, 1,400µm, 1,500µm, 2,000µm, 2,500µm, 3,000µm, 3,500µm, 4,000µm, 4,500µm, 5,000µm, 5,500µm, 6,000µm, 6,500µm, 7,000µm, 7,500µm, 8,000µm, 8,500µm, 9,000µm, 9,500µm or 10,000µm. Research has found that, when the length of the channel is 30µm-10,000µm, it can effectively monitor the monitored object and provide good wearing comfort; when the length of the channel is less than 30µm, there is a risk that the analyte sensor cannot effectively contact the body fluid, thereby leading to the inability to monitor; when the length of the channel is greater than 10,000µm, due to excessive implantation, the wearer is prone to bleeding and pain, which affects the wearing experience.

According to the embodiments of the present application, the channel can be obtained through printing, engraving, etching, inkjet printing, electrohydrodynamic (EHD) printing, dispensing, spraying, chemical vapor deposition (CVD), physical vapor deposition (PVD), magnetron sputtering, molecular beam epitaxy, electrochemical deposition, and evaporation deposition and other methods.

### <structure of the sensor>

According to the embodiments of the present application, the analyte sensor is a four-electrode system, and the combination of four electrodes can stabilize the potential.

According to the embodiments of the present application, the analyte sensor can continuously monitor for a period of 7-360 days. The analyte sensor can continuously monitor two analytes/physiological indicators, such as monitoring at least once a day, such as 1-10 times; or monitoring at least once per hour, such as 1-10 times; or monitoring at least once per minute, such as 1-30 times; or monitoring at least once per second, such as 1-10 times.

According to the embodiments of the present application, the analyte sensor has an L-shape. On the one hand, the L-shaped analyte sensor can be adapted to the auxiliary device to better complete analysis work; on the other hand, the L-shape can effectively prevent sensor damage during implantation, increasing the probability of successful sensor implantation.

According to the embodiments of the present application, the analyte sensor has an L-shape, which includes a first side and a second side, and an angle of 45°-135° is formed at the connection between the first side and the second side, preferably 88°-98°.

According to the embodiments of the present application, there are two contact areas and two electrode areas on a first surface of the analyte sensor, and there is one contact area and one electrode area on a second surface of the analyte sensor opposite to the first surface. Furthermore, the electrode area is located at the first side of the L-shaped analyte sensor, and the contact area is located at the second side of the sensor.

According to the embodiments of the present application, there are two contact areas and two electrode areas on a first surface of the analyte sensor, and there are two contact areas and two electrode areas on a second surface of the analyte sensor opposite to the first surface.

According to the embodiments of the present application, there are three contact areas and three electrode areas on a first surface of the analyte sensor, and there is one contact area and one electrode area on a second surface of the analyte sensor opposite to the first surface. Furthermore, the electrode area is located at the first side of the L-shaped analyte sensor, and the contact area is located at the second side of the sensor.

### <protruding structure>

According to the embodiments of the present application, a protruding structure 118 is formed at the connection between the first side and the second side. Preferably, the outer edge of the connection between the first side and the second side forms a protruding structure, and the provision of the protruding structure can adapt the sensor to the auxiliary device during implantation, and can make the sensor smoother during implantation.

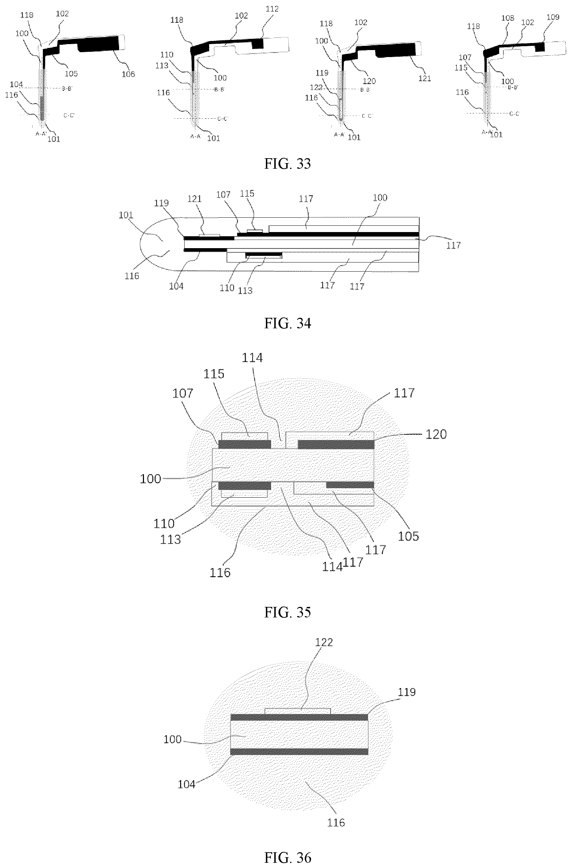

According to the embodiments of the present application, the protruding structure can be semicircular, triangular, square, or polygonal, as shown in FIG. 34.

According to the embodiments of the present application, there may be one protruding structure, and may also be more protruding structures, as shown in FIG. 34.

### <dielectric layer>

According to the embodiments of the present application, the dielectric layer is provided on the surface of the first working electrode cable of the first working electrode to protect the working electrode from damage; the dielectric layer is provided on the surface of the second working electrode cable of the second working electrode to protect the working electrode from damage; the dielectric layer is provided on the surface of the reference electrode to protect the reference electrode from damage; the dielectric layer is provided on the surface of the counter electrode cable of the counter electrode to protect the counter electrode from damage.

According to the embodiments of the present application, the material forming the dielectric layer comprises at least one of plastic (such as polyethylene terephthalate PET, polyimide PI, polyvinyl chloride PVC, or polyethylene PE), polydimethylsiloxane PDMS, ceramics, glass, paper, and textiles.

According to the embodiments of the present application, the dielectric layer is formed on the reference electrode, working electrode cable or counter electrode cable through process methods including but not limited to adsorption, printing, inkjet printing, electrohydrodynamic (EHD) printing, dispensing, spraying, chemical vapor deposition (CVD), physical vapor deposition (PVD), magnetron sputtering, molecular beam epitaxy, electrochemical deposition, and evaporation deposition.

According to the embodiments of the present application, the thickness of the dielectric layer is 1µm-1,000µm, preferably 10µm-500µm, such as 10µm, 20µm, 50µm, 80µm, 100µm, 120µm, 150µm, 180µm, 200µm, 220µm, 250µm, 260µm, 280µm, 300µm, 320µm, 350µm, 380µm, 400µm, 420µm, 450µm, 480µm, 500µm, 600µm, 700µm, 800µm, 900µm or 1,000µm.

In all sensor configurations disclosed herein, various electrodes may be separated from each other by channels.

### <protective layer>

According to the embodiments of the present application, the analyte sensor further comprises a protective layer 116, which is provided on the outermost layer of the sensor.

According to the embodiments of the present application, the protective layer may be a single-layer structure or a multi-layer structure.

According to the embodiments of the present application, the protective layer may be configured to limit flux. The protective layer may be configured to prevent biofouling. The protective layer may be configured to shield environmental noise. The protective layer may be configured to protect internal components/elements from corrosion and damage.

According to the embodiments of the present application, the protective layer can be in the form of gel or solid. According to the embodiments of the present application, the protective layer may be a component, such as a filter.

According to the embodiments of the present application, the material forming the protective layer may be ethylene homopolymer or ethylene copolymer.

According to the embodiments of the present application, the thickness of the protective layer is 0.5µm-2000µm, preferably, the thickness of the protective layer is 2µm-1000µm, such as 5µm, 10µm, 50µm, 80µm, 100µm, 150µm, 200µm, 250µm, 300µm, 350µm, 400µm, 450µm, 500µm, 600µm, 700µm, 800µm, 900µm or 1,000µm. According to the embodiments of the present application, the protective layer is modified onto the sensor through processes including but not limited to dispensing, spin coating, spray coating, dip coating, blade coating, nesting, wrapping, printing, inkjet printing, electrohydrodynamic (EHD) printing, electrospinning, dispensing, spraying, chemical vapor deposition (CVD), physical vapor deposition (PVD), magnetron sputtering, molecular beam epitaxy, electrochemical deposition, and evaporation deposition.

### <instructions for analyte sensors>

According to the embodiments of the present application, the monitoring technology of the analyte sensor for blood glucose can be any one of the first generation, second generation, third generation, or fourth generation. Specifically, different testing technologies can be used to monitor blood glucose based on the different working electrode materials and sensing layer materials.

According to the embodiments of the present application, the analyte sensor comprises a first working electrode, a second working electrode, a reference electrode, and a counter electrode. The analyte sensor is implanted into the body through minimally invasive means using an auxiliary device. Specifically, the front end and partial middle end of the analyte sensor (i.e., the position below the protruding structure, one side of the L-shape) are implanted into the body. The first working electrode and the counter electrode form a current circuit, and the first working electrode and the reference electrode form a voltage circuit; the second working electrode and the counter electrode form a current circuit, and the second working electrode and the reference electrode form a voltage circuit, so that the first sensing layer located on the first working electrode reacts with the first analyte to generate an electrochemical signal, thereby monitoring the first analyte, and the second sensing layer located on the second working electrode reacts with the second analyte to generate an electrochemical signal, thereby monitoring the second analyte; furthermore, there is a corresponding relationship between the intensity of the generated electrochemical signal and the concentration of the analyte, that is, the higher the concentration of the analyte, the stronger the electrochemical properties generated.

According to the embodiments of the present application, the analyte sensor can test the first analyte and the second analyte through one analysis method, or can test the first analyte and the second analyte separately through two analysis methods. For example, the analysis methods include electrochemical+electrochemical, electrochemical+mechanical, electrochemical+mechanistic, electrochemical+electrical, electrochemical+thermal, electrochemical+optical, electrochemical+acoustic, electrochemical+electromagnetic, and other methods. According to the embodiments of the present application, the analyte sensor may contain enzymes or not contain enzymes, or a combination of enzymes and no enzymes. For example, the analyte sensor can test/monitor blood glucose+uric acid, lactate, ketones, cholesterol, triglycerides, heart rate, blood pressure, Na⁺, Ka⁺, Ga²⁺, Fe²⁺, Mg²⁺, etc. through enzymatic or non-enzymatic sensor technology.

According to the embodiments of the present application, the analyte sensor comprises a first working electrode, a second working electrode, a reference electrode, and a counter electrode. The analyte sensor is implanted into the body through minimally invasive means using an auxiliary device. Specifically, the front end and partial middle end of the analyte sensor (i.e., the position below the protruding structure, one side of the L-shape) are implanted into the body. The first working electrode, the second working electrode and the counter electrode form a current circuit, and the first working electrode, the second working electrode and the reference electrode form a voltage circuit, so that the first sensing layer located on the first working electrode reacts with the first analyte to generate an electrochemical signal, thereby monitoring the first analyte, and meanwhile the second sensing layer located on the second working electrode reacts with the second analyte to generate an electrochemical signal, thereby monitoring the second analyte; furthermore, there is a corresponding relationship between the intensity of the generated electrochemical signal and the concentration of the analyte, that is, the higher the concentration of the analyte, the stronger the electrochemical properties generated.

### <testing method for determining analytes>

A testing method for determining analytes, comprising:
1) the analyte sensor is exposed to a fluid containing different concentrations of a first analyte and a second analyte, and voltage is applied to the first working electrode of the sensor, in order to obtain first electrochemical signals with different signal intensities; the signal intensity of the first electrochemical signal is proportional to the concentration of the first analyte in the fluid; voltage is applied to the second working electrode in the sensor to obtain second electrochemical signals with different signal intensities, and the signal intensity of the second electrochemical signal is proportional to the concentration of the second analyte in the fluid;
2) the signal intensity of the first electrochemical signal is associated with the concentration of the first analyte in the fluid, and a standard curve of the signal intensity of the first electrochemical signal and the concentration of the first analyte is drawn; the signal intensity of the second electrochemical signal is associated with the concentration of the second analyte in the fluid, and a standard curve of the signal intensity of the second electrochemical signal and the concentration of the second analyte is drawn;
3) the analyte sensor is exposed to a fluid containing the monitored first analyte and second analyte, and voltage is applied to the first working electrode of the sensor, in order to obtain a first electrochemical signal, thereby obtaining the concentration of the monitored first analyte in the fluid based on the signal intensity of the obtained first electrochemical signal; voltage is applied to the second working electrode in the sensor to obtain a second electrochemical signal, thereby obtaining the concentration of the monitored second analyte in the fluid based on the signal intensity of the obtained second electrochemical signal.

The present application will be further clarified below in combination with the specific embodiments. It should be understood that the following embodiments are only illustrative and explanatory of the present application, and should not be interpreted as limiting the scope of protection of the present application. All technologies implemented based on the above content of the present application are covered within the scope of protection intended by the present application.

Unless otherwise specified, experimental methods applied in the following embodiments are conventional methods; unless otherwise specified, all the materials and reagents applied in the following embodiments are commercially available.

### Embodiment 1

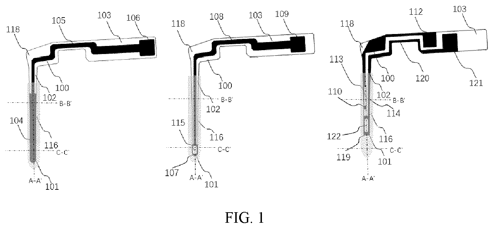

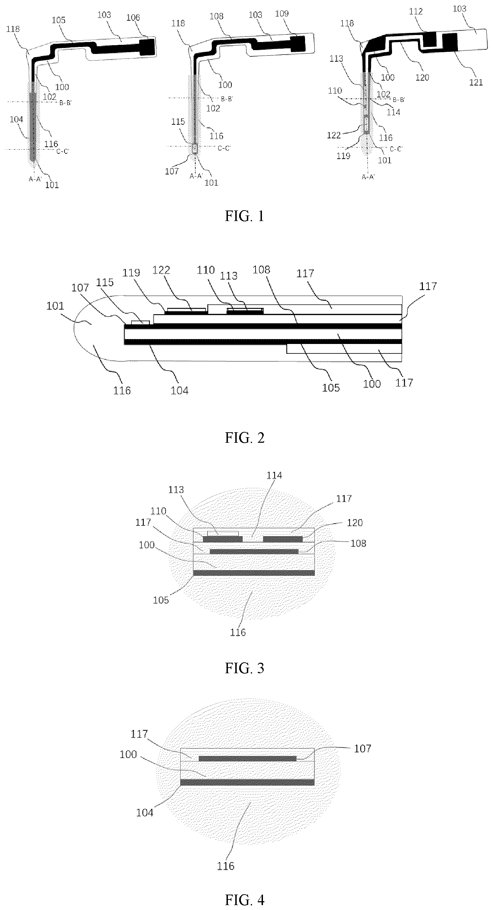

The structure of the sensor electrode structure A in Embodiment 1 is as shown in FIGs. 1-4. FIG. 1 is a view of the sensor electrode structure A in Embodiment 1, where the left figure is a back view of the sensor electrode structure A, the middle figure is a front view of the second layer of the sensor electrode structure A, and the right figure is a front view of the first layer of the sensor electrode structure A. It can be seen from FIG. 1 that the sensor electrode structure A comprises a substrate 100, a reference electrode, a counter electrode, a first working electrode, and a second working electrode: the substrate 100 has an L-shaped structure and the substrate 100 comprises a front end 101, a middle end 102, and a distal end 103; the reference electrode, the first working electrode, and the second working electrode are located on a first surface of the substrate 100 (i.e., the front of the sensor electrode structure A), and the counter electrode is located on a second surface of the substrate 100 opposite to the first surface (i.e., the back of the sensor electrode structure A); specifically, the first working electrode is provided on the surface of the substrate 100, and the reference electrode is stacked on the surface of the first working electrode and separated by a dielectric layer 117; the second working electrode is stacked on the surface of the first working electrode and separated by a dielectric layer 117, the second working electrode and the reference electrode are spatially separated by a channel, and all components of the second working electrode and the reference electrode are on the same plane.

The first working electrode comprises a first working electrode layer 107 located at the front end 101 of the substrate, a first working electrode cable 108 located at the middle end 102 of the substrate, and a first working electrode contact 109 located at the distal end 103 of the substrate; the first working electrode comprises a first sensing layer 115, which is located on the first working electrode layer 107; the second working electrode comprises a second working electrode layer 119 located at the front end 101 of the substrate, a second working electrode cable 120 located at the middle end 102 of the substrate, and a second working electrode contact 121 located at the distal end 103 of the substrate; the second working electrode comprises a second sensing layer 122, which is located on the second working electrode layer 119; the reference electrode comprises a reference electrode layer 113 and a reference electrode cable 110 located at the middle end 102 of the substrate, a reference electrode contact 112 located at the distal end 103 of the substrate, and the reference electrode layer 113 is provided on the reference electrode cable 110; the counter electrode comprises a counter electrode layer 104 located at the front end 101 of the substrate, a counter electrode cable 105 located at the middle end 102 of the substrate, and a counter electrode contact 106 located at the distal end 103 of the substrate.

The sensor electrode structure A comprises a channel 114 located between the second working electrode cable 120 of the second working electrode and the reference electrode layer 113, and the second working electrode and the reference electrode are separated by the channel 114; the width of the channel 114 is adjustable within the range of 1µm-280µm, and the length of the channel is adjustable within the range of 30µm-10,000µm. The sensor electrode structure A has an L-shape, which includes a first side and a second side, and an angle of 85°-95° is formed at the connection between the first side and the second side. A protruding structure 118 is formed at the outer edge of the connection between the first side and the second side. The sensor electrode structure A comprises a protective layer 116, which is provided outside of the sensor.

FIG. 2 is a structural diagram of the section A-A' of the sensor electrode structure A in Embodiment 1. FIG. 3 is a structural diagram of the section B-B' of the sensor electrode structure A in Embodiment 1. FIG. 4 is a structural diagram of the section C-C' of the sensor electrode structure A in Embodiment 1. It can be seen from FIGs. 2-4 that, the first working electrode is provided on the surface of the substrate 100, and the reference electrode is stacked on the surface of the first working electrode and separated by a dielectric layer 117; the second working electrode is stacked on the surface of the first working electrode and separated by a dielectric layer 117, the second working electrode and the reference electrode are spatially separated by a channel, and all components of the second working electrode and the reference electrode are on the same plane; the first working electrode is closer to the front end 101 of the substrate than the reference electrode and the second working electrode.

### Embodiment 2

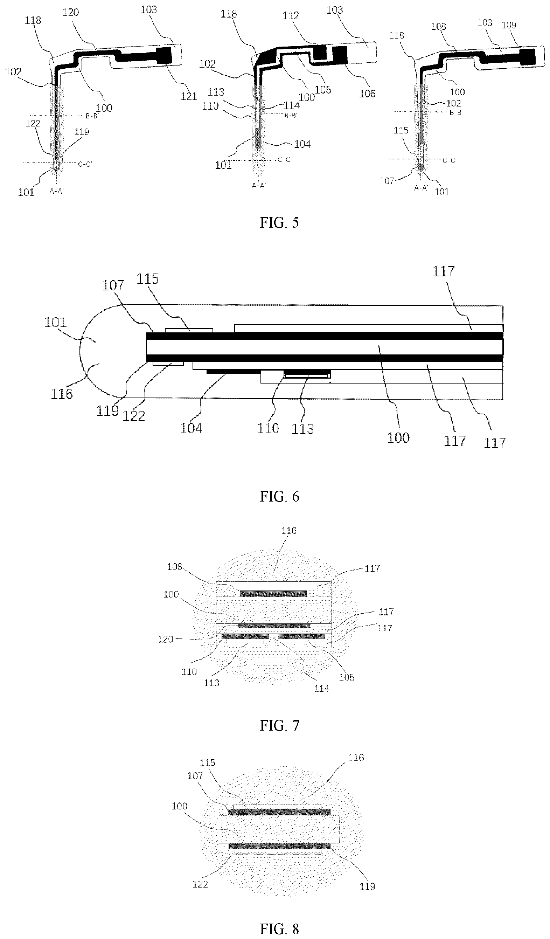

The structure of the sensor electrode structure B in Embodiment 2 is as shown in FIGs. 5-8. FIG. 5 is a view of the sensor electrode structure B in Embodiment 2, where the left figure is a back view of the second layer of the sensor electrode structure B, the middle figure is a back view of the first layer of the sensor electrode structure B, and the right figure is a front view of the sensor electrode structure B. It can be seen from FIG. 5 that the sensor electrode structure B comprises a substrate 100, a reference electrode, a counter electrode, a first working electrode, and a second working electrode: the substrate 100 has an L-shaped structure and the substrate 100 comprises a front end 101, a middle end 102, and a distal end 103; the reference electrode, the counter electrode, and the second working electrode are located on a second surface of the substrate 100 opposite to the first surface (i.e., the back of the sensor electrode structure B), and the first working electrode is located on a first surface of the substrate 100 (i.e., the front of the sensor electrode structure B); specifically, the second working electrode is provided on the surface of the substrate 100, and the reference electrode is stacked on the surface of the second working electrode and separated by a dielectric layer 117; the counter electrode is stacked on the surface of the second working electrode and separated by a dielectric layer 117, the counter electrode and the reference electrode are spatially separated by a channel, and all components of the counter electrode and the reference electrode are on the same plane.

The first working electrode comprises a first working electrode layer 107 located at the front end 101 of the substrate, a first working electrode cable 108 located at the middle end 102 of the substrate, and a first working electrode contact 109 located at the distal end 103 of the substrate; the first working electrode comprises a first sensing layer 115, which is located on the first working electrode layer 107; the second working electrode comprises a second working electrode layer 119 located at the front end 101 of the substrate, a second working electrode cable 120 located at the middle end 102 of the substrate, and a second working electrode contact 121 located at the distal end 103 of the substrate; the second working electrode comprises a second sensing layer 122, which is located on the second working electrode layer 119; the reference electrode comprises a reference electrode layer 113 and a reference electrode cable 110 located at the middle end 102 of the substrate, a reference electrode contact 112 located at the distal end 103 of the substrate, and the reference electrode layer 113 is provided on the reference electrode cable 110; the counter electrode comprises a counter electrode layer 104 located at the front end 101 of the substrate, a counter electrode cable 105 located at the middle end 102 of the substrate, and a counter electrode contact 106 located at the distal end 103 of the substrate.

The sensor electrode structure B comprises a channel 114 located between the counter electrode cable 105 and the reference electrode layer 113, and the counter electrode and the reference electrode are separated by the channel 114; the width of the channel 114 is adjustable within the range of 1µm-280µm, and the length of the channel is adjustable within the range of 30µm-10,000µm. The sensor electrode structure B has an L-shape, which includes a first side and a second side, and an angle of 85°-95° is formed at the connection between the first side and the second side. A protruding structure 118 is formed at the outer edge of the connection between the first side and the second side. The sensor electrode structure B comprises a protective layer 116, which is provided outside of the sensor.

FIG. 6 is a structural diagram of the section A-A' of the sensor electrode structure B in Embodiment 2. FIG. 7 is a structural diagram of the section B-B' of the sensor electrode structure B in Embodiment 2. FIG. 8 is a structural diagram of the section C-C' of the sensor electrode structure B in Embodiment 2. It can be seen from FIGs. 6-8 that, the second working electrode is provided on the surface of the substrate 100, and the reference electrode is stacked on the surface of the second working electrode and separated by a dielectric layer 117; the counter electrode is stacked on the surface of the second working electrode and separated by a dielectric layer 117, the counter electrode and the reference electrode are spatially separated by a channel, and all components of the counter electrode and the reference electrode are on the same plane; the second working electrode is closer to the front end 101 of the substrate than the reference electrode and the counter electrode.

### Embodiment 3

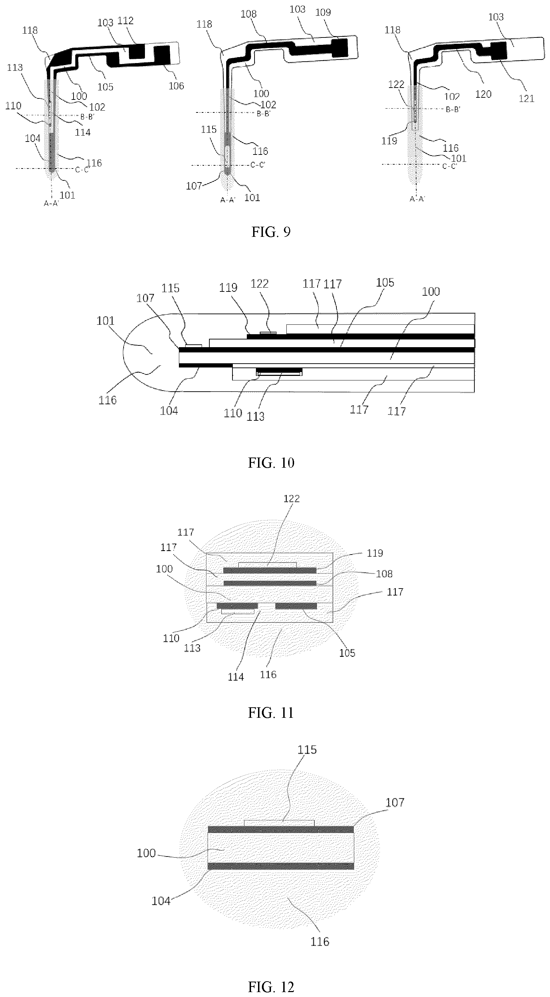

The structure of the sensor electrode structure C in Embodiment 3 is as shown in FIGs. 9-12. FIG. 9 is a view of the sensor electrode structure C in Embodiment 3, where the left figure is a back view of the sensor electrode structure C, the middle figure is a front view of the second layer of the sensor electrode structure C, and the right figure is a front view of the first layer of the sensor electrode structure C. It can be seen from FIG. 9 that the sensor electrode structure C comprises a substrate 100, a reference electrode, a counter electrode, a first working electrode, and a second working electrode: the substrate 100 has an L-shaped structure and the substrate 100 comprises a front end 101, a middle end 102, and a distal end 103; the reference electrode and the counter electrode are located on a second surface of the substrate 100 opposite to the first surface (i.e., the back of the sensor electrode structure C), and the first working electrode and the second working electrode are located on the first surface of the substrate 100 (i.e., the front of the sensor electrode structure C); specifically, the first working electrode is provided on the first surface of the substrate 100, and the second working electrode is stacked on the surface of the first working electrode and separated by a dielectric layer 117; the first working electrode is closer to the front end 101 of the substrate than the second working electrode; the counter electrode and the reference electrode are provided on the second surface of the substrate opposite to the first surface, and spatially separated by a channel, and all components of the counter electrode and the reference electrode are on the same plane.

The first working electrode comprises a first working electrode layer 107 located at the front end 101 of the substrate, a first working electrode cable 108 located at the middle end 102 of the substrate, and a first working electrode contact 109 located at the distal end 103 of the substrate; the first working electrode comprises a first sensing layer 115, which is located on the first working electrode layer 107; the second working electrode comprises a second working electrode layer 119 and a second working electrode cable 120 located at the middle end 102 of the substrate, and a second working electrode contact 121 located at the distal end 103 of the substrate; the second working electrode comprises a second sensing layer 122, which is located on the second working electrode layer 119; the reference electrode comprises a reference electrode layer 113 and a reference electrode cable 110 located at the middle end 102 of the substrate, a reference electrode contact 112 located at the distal end 103 of the substrate, and the reference electrode layer 113 is provided on the reference electrode cable 110; the counter electrode comprises a counter electrode layer 104 located at the front end 101 of the substrate, a counter electrode cable 105 located at the middle end 102 of the substrate, and a counter electrode contact 106 located at the distal end 103 of the substrate.

The sensor electrode structure C comprises a channel 114 located between the counter electrode cable 105 and the reference electrode layer 113, and the counter electrode and the reference electrode are separated by the channel 114; the width of the channel 114 is adjustable within the range of 1µm-280µm, and the length of the channel is adjustable within the range of 30µm-10,000µm. The sensor electrode structure C has an L-shape, which includes a first side and a second side, and an angle of 85°-95° is formed at the connection between the first side and the second side. A protruding structure 118 is formed at the outer edge of the connection between the first side and the second side. The sensor electrode structure C comprises a protective layer 116, which is provided outside of the sensor.

FIG. 10 is a structural diagram of the section A-A' of the sensor electrode structure C in Embodiment 3. FIG. 11 is a structural diagram of the section B-B' of the sensor electrode structure C in Embodiment 3. FIG. 12 is a structural diagram of the section A-A' of the sensor electrode structure C in Embodiment 3. It can be seen from FIGs. 10-12 that, the first working electrode is provided on the first surface of the substrate 100, and the second working electrode is stacked on the surface of the first working electrode and separated by a dielectric layer 117; the first working electrode is closer to the front end 101 of the substrate than the second working electrode; the counter electrode and the reference electrode are provided on the second surface of the substrate 100 opposite to the first surface, and spatially separated by a channel, and all components of the counter electrode and the reference electrode are on the same plane.

### Embodiment 4

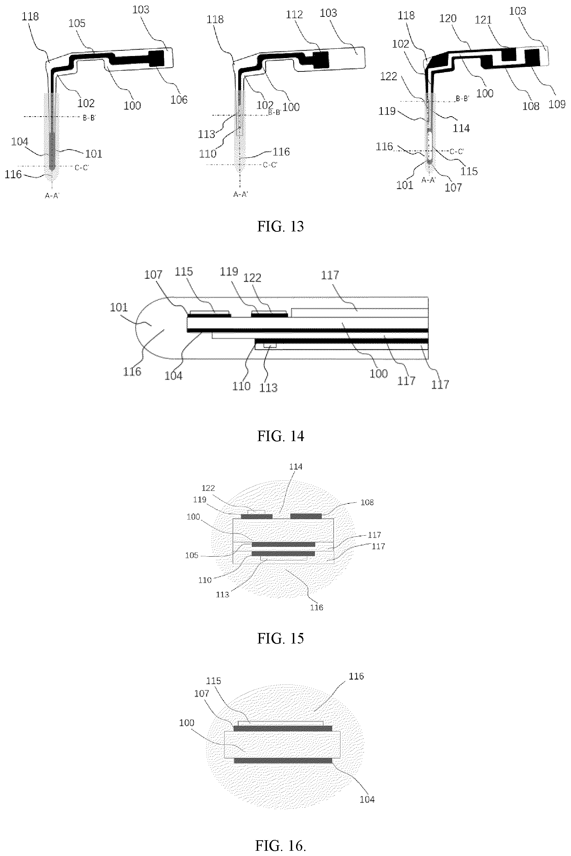

The structure of the sensor electrode structure D in Embodiment 4 is as shown in FIGs. 13-16. FIG. 13 is a view of the sensor electrode structure D in Embodiment 4, where the left figure is a back view of the second layer of the sensor electrode structure D, the middle figure is a back view of the first layer of the sensor electrode structure D, and the right figure is a front view of the sensor electrode structure D. It can be seen from FIG. 13 that the sensor electrode structure D comprises a substrate 100, a reference electrode, a counter electrode, a first working electrode, and a second working electrode: the substrate 100 has an L-shaped structure and the substrate 100 comprises a front end 101, a middle end 102, and a distal end 103; the reference electrode and the counter electrode are located on a second surface of the substrate 100 opposite to the first surface (i.e., the back of the sensor electrode structure D), and the first working electrode and the second working electrode are located on a first surface of the substrate 100 (i.e., the front of the sensor electrode structure D); specifically, the first working electrode and the second working electrode are provided on the first surface of the substrate 100, and the first working electrode and the second working electrode are spatially separated by a channel, and all components of the first working electrode and the second working electrode are on the same plane; the counter electrode is provided on the second surface of the substrate 100 opposite to the first surface, and the reference electrode is stacked on the surface of the counter electrode and separated by a dielectric layer 117; the counter electrode is closer to the front end 101 of the substrate than the reference electrode.

The first working electrode comprises a first working electrode layer 107 located at the front end 101 of the substrate, a first working electrode cable 108 located at the middle end 102 of the substrate, and a first working electrode contact 109 located at the distal end 103 of the substrate; the first working electrode comprises a first sensing layer 115, which is located on the first working electrode layer 107; the second working electrode comprises a second working electrode layer 119 and a second working electrode cable 120 located at the middle end 102 of the substrate, and a second working electrode contact 121 located at the distal end 103 of the substrate; the second working electrode comprises a second sensing layer 122, which is located on the second working electrode layer 119; the reference electrode comprises a reference electrode layer 113 and a reference electrode cable 110 located at the middle end 102 of the substrate, a reference electrode contact 112 located at the distal end 103 of the substrate, and the reference electrode layer 113 is provided on the reference electrode cable 110; the counter electrode comprises a counter electrode layer 104 located at the front end 101 of the substrate, a counter electrode cable 105 located at the middle end 102 of the substrate, and a counter electrode contact 106 located at the distal end 103 of the substrate.

The sensor electrode structure D comprises a channel 114 located between the first working electrode cable 108 and the second working electrode layer 119, and the first working electrode and the second working electrode are separated by the channel 114; the width of the channel 114 is adjustable within the range of 1µm-280µm, and the length of the channel is adjustable within the range of 30µm-10,000µm. The sensor electrode structure D has an L-shape, which includes a first side and a second side, and an angle of 85°-95° is formed at the connection between the first side and the second side. A protruding structure 118 is formed at the outer edge of the connection between the first side and the second side. The sensor electrode structure D comprises a protective layer 116, which is provided outside of the sensor.

FIG. 14 is a structural diagram of the section A-A' of the sensor electrode structure D in Embodiment 4. FIG. 15 is a structural diagram of the section B-B' of the sensor electrode structure D in Embodiment 4. FIG. 16 is a structural diagram of the section C-C' of the sensor electrode structure D in Embodiment 4. It can be seen from FIGs. 14-16 that, the first working electrode and the second working electrode are provided on the first surface of the substrate 100, and the first working electrode and the second working electrode are spatially separated by a channel, and all components of the first working electrode and the second working electrode are on the same plane; the counter electrode is provided on the second surface of the substrate 100 opposite to the first surface, and the reference electrode is stacked on the surface of the counter electrode and separated by a dielectric layer 117; the counter electrode is closer to the front end 101 of the substrate than the reference electrode.

### Embodiment 5

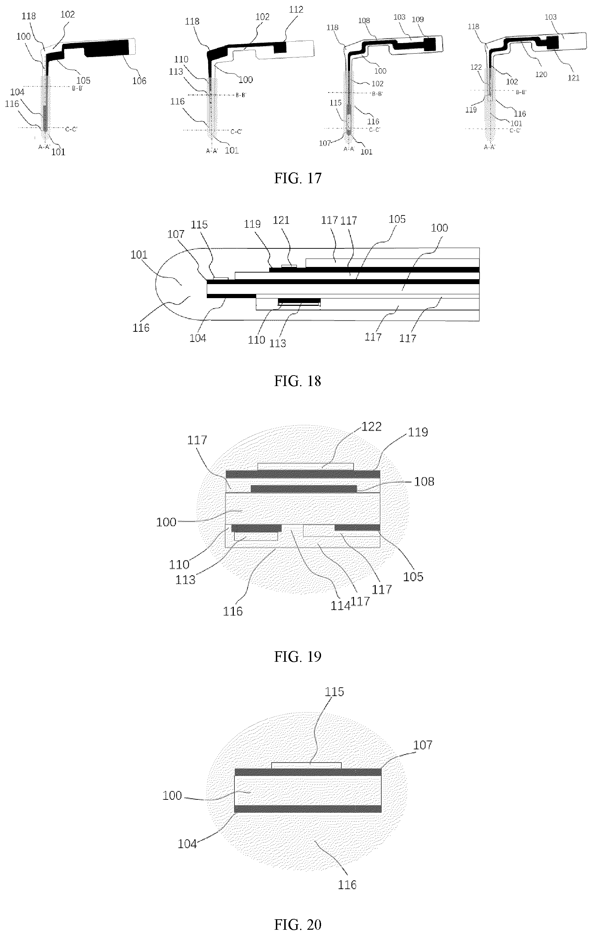

The structure of the sensor electrode structure E in Embodiment 5 is as shown in FIGs. 17-20. FIG. 17 is a view of the sensor electrode structure E in Embodiment 5, where the first left figure is a back view of the second layer of the sensor electrode structure E, and the second left figure is a back view of the first layer of the sensor electrode structure E; the first right figure is a front view of the first layer of the sensor electrode structure E, and the second right figure is a front view of the second layer of the sensor electrode structure E. It can be seen from FIG. 17 that the sensor electrode structure E comprises a substrate 100, a reference electrode, a counter electrode, a first working electrode, and a second working electrode: the substrate 100 has an L-shaped structure and the substrate 100 comprises a front end 101, a middle end 102, and a distal end 103; the reference electrode and the counter electrode are located on a second surface of the substrate 100 opposite to the first surface (i.e., the back of the sensor electrode structure E), and the first working electrode and the second working electrode are located on a first surface of the substrate 100 (i.e., the front of the sensor electrode structure E); specifically, the first working electrode is provided on the first surface of the substrate 100, and the second working electrode is stacked on the surface of the first working electrode and separated by a dielectric layer 117; the first working electrode is closer to the front end 101 of the substrate than the second working electrode; the counter electrode and the reference electrode are provided on the second surface of the substrate opposite to the first surface, and spatially separated by a channel, and some components of the counter electrode and the reference electrode are on the same plane, while some components are on different planes.

The first working electrode comprises a first working electrode layer 107 located at the front end 101 of the substrate, a first working electrode cable 108 located at the middle end 102 of the substrate, and a first working electrode contact 109 located at the distal end 103 of the substrate; the first working electrode comprises a first sensing layer 115, which is located on the first working electrode layer 107; the second working electrode comprises a second working electrode layer 119 and a second working electrode cable 120 located at the middle end 102 of the substrate, and a second working electrode contact 121 located at the distal end 103 of the substrate; the second working electrode comprises a second sensing layer 122, which is located on the second working electrode layer 119; the reference electrode comprises a reference electrode layer 113 and a reference electrode cable 110 located at the middle end 102 of the substrate, a reference electrode contact 112 located at the distal end 103 of the substrate, and the reference electrode layer 113 is provided on the reference electrode cable 110; the counter electrode comprises a counter electrode layer 104 located at the front end 101 of the substrate, a counter electrode cable 105 located at the middle end 102 of the substrate, and a counter electrode contact 106 located at the distal end 103 of the substrate.

The sensor electrode structure E comprises a channel 114 located between the counter electrode cable 105 and the reference electrode layer 113, and the counter electrode and the reference electrode are separated by the channel 114; the width of the channel 114 is adjustable within the range of 1µm-280µm, and the length of the channel is adjustable within the range of 30µm-10,000µm. The sensor electrode structure E has an L-shape, which includes a first side and a second side, and an angle of 85°-95° is formed at the connection between the first side and the second side. A protruding structure 118 is formed at the outer edge of the connection between the first side and the second side. The sensor electrode structure E comprises a protective layer 116, which is provided outside of the sensor.

FIG. 18 is a structural diagram of the section A-A' of the sensor electrode structure E in Embodiment 5. FIG. 19 is a structural diagram of the section B-B' of the sensor electrode structure E in Embodiment 5. FIG. 20 is a structural diagram of the section C-C' of the sensor electrode structure E in Embodiment 5. It can be seen from FIGs. 18-20 that, the first working electrode is provided on the first surface of the substrate 100, and the second working electrode is stacked on the surface of the first working electrode and separated by a dielectric layer 117; the first working electrode is closer to the front end 101 of the substrate than the second working electrode; the counter electrode and the reference electrode are provided on the second surface of the substrate opposite to the first surface, and spatially separated by a channel, and some components of the counter electrode and the reference electrode are on the same plane, while some components are on different planes.

### Embodiment 6

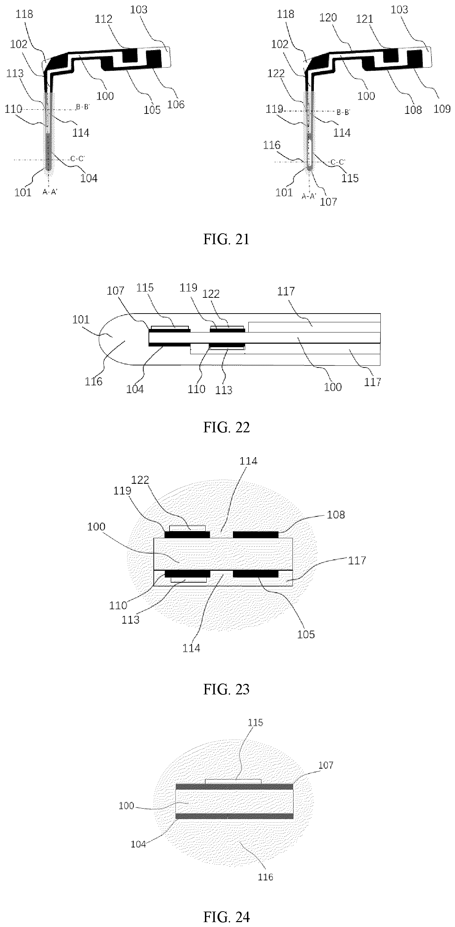

The structure of the sensor electrode structure F in Embodiment 6 is as shown in FIGs. 21-24. FIG. 21 is a view of the sensor electrode structure F in Embodiment 6, where the left figure is a back view of the sensor electrode structure F and the right figure is a front view of the sensor electrode structure F. It can be seen from FIG. 21 that the sensor electrode structure F comprises a substrate 100, a reference electrode, a counter electrode, a first working electrode, and a second working electrode: the substrate 100 has an L-shaped structure and the substrate 100 comprises a front end 101, a middle end 102, and a distal end 103; the reference electrode and the counter electrode are located on a second surface of the substrate 100 opposite to the first surface (i.e., the back of the sensor electrode structure F), and the first working electrode and the second working electrode are located on a first surface of the substrate 100 (i.e., the front of the sensor electrode structure F); specifically, the first working electrode and the second working electrode are provided on the first surface of the substrate 100, and spatially separated by a channel, and all components of the first working electrode and the second working electrode are on the same plane; the counter electrode and the reference electrode are provided on the second surface of the substrate 100 opposite to the first surface, the counter electrode and the reference electrode are spatially separated by a channel, and all components of the counter electrode and the reference electrode are on the same plane.

The first working electrode comprises a first working electrode layer 107 located at the front end 101 of the substrate, a first working electrode cable 108 located at the middle end 102 of the substrate, and a first working electrode contact 109 located at the distal end 103 of the substrate; the first working electrode comprises a first sensing layer 115, which is located on the first working electrode layer 107; the second working electrode comprises a second working electrode layer 119 and a second working electrode cable 120 located at the middle end 102 of the substrate, and a second working electrode contact 121 located at the distal end 103 of the substrate; the second working electrode comprises a second sensing layer 122, which is located on the second working electrode layer 119; the reference electrode comprises a reference electrode layer 113 and a reference electrode cable 110 located at the middle end 102 of the substrate, a reference electrode contact 112 located at the distal end 103 of the substrate, and the reference electrode layer 113 is provided on the reference electrode cable 110; the counter electrode comprises a counter electrode layer 104 located at the front end 101 of the substrate, a counter electrode cable 105 located at the middle end 102 of the substrate, and a counter electrode contact 106 located at the distal end 103 of the substrate.

The sensor electrode structure F comprises a channel 114 located between the counter electrode cable 105 and the reference electrode layer 113, and the counter electrode and the reference electrode are separated by the channel 114; the width of the channel 114 is adjustable within the range of 1µm-280µm, and the length of the channel is adjustable within the range of 30µm-10,000µm. The sensor electrode structure F also comprises a channel 114 located between the first working electrode cable 108 and the second working electrode layer 119, and the first working electrode and the second working electrode are separated by the channel 114; the width of the channel 114 is adjustable within the range of 1µm-280µm, and the length of the channel is adjustable within the range of 30µm-10,000µm. The sensor electrode structure F has an L-shape, which includes a first side and a second side, and an angle of 85°-95° is formed at the connection between the first side and the second side. A protruding structure 118 is formed at the outer edge of the connection between the first side and the second side. The sensor electrode structure F comprises a protective layer 116, which is provided outside of the sensor.

FIG. 22 is a structural diagram of the section A-A' of the sensor electrode structure F in Embodiment 6. FIG. 23 is a structural diagram of the section B-B' of the sensor electrode structure F in Embodiment 6. FIG. 24 is a structural diagram of the section C-C' of the sensor electrode structure F in Embodiment 6. It can be seen from FIGs. 22-24 that, the first working electrode and the second working electrode are provided on the first surface of the substrate 100, and the first working electrode and the second working electrode are spatially separated by a channel, and all components of the first working electrode and the second working electrode are on the same plane; the counter electrode and the reference electrode are provided on the second surface of the substrate 100 opposite to the first surface, and spatially separated by a channel, and all components of the counter electrode and the reference electrode are on the same plane.

### Embodiment 7

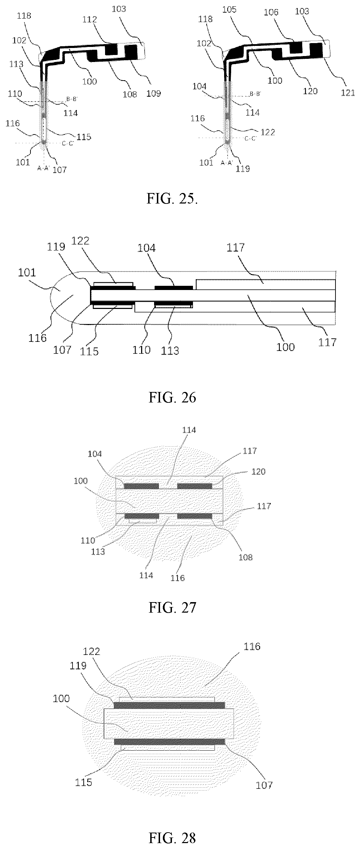

The structure of the sensor electrode structure G in Embodiment 7 is as shown in FIGs. 25-28. FIG. 25 is a view of the sensor electrode structure G in Embodiment 7, where the left figure is a back view of the sensor electrode structure G and the right figure is a front view of the sensor electrode structure G. It can be seen from FIG. 25 that the sensor electrode structure G comprises a substrate 100, a reference electrode, a counter electrode, a first working electrode, and a second working electrode: the substrate 100 has an L-shaped structure and the substrate 100 comprises a front end 101, a middle end 102, and a distal end 103; the reference electrode and the first working electrode are located on a second surface of the substrate 100 opposite to the first surface (i.e., the back of the sensor electrode structure G), and the counter electrode and the second working electrode are located on a first surface of the substrate 100 (i.e., the front of the sensor electrode structure G); specifically, the first working electrode and the reference electrode are provided on the first surface of the substrate 100, and spatially separated by a channel, and all components of the first working electrode and the reference electrode are on the same plane; the counter electrode and the second working electrode are provided on the second surface of the substrate 100 opposite to the first surface, the counter electrode and the second working electrode are spatially separated by a channel, and all components of the counter electrode and the second working electrode are on the same plane. The first working electrode comprises a first working electrode layer 107 located at the front end 101 of the substrate, a first working electrode cable 108 located at the middle end 102 of the substrate, and a first working electrode contact 109 located at the distal end 103 of the substrate; the first working electrode comprises a first sensing layer 115, which is located on the first working electrode layer 107; the second working electrode comprises a second working electrode layer 119 located at the front end 101 of the substrate, a second working electrode cable 120 located at the middle end 102 of the substrate, and a second working electrode contact 121 located at the distal end 103 of the substrate; the second working electrode comprises a second sensing layer 122, which is located on the second working electrode layer 119; the reference electrode comprises a reference electrode layer 113 and a reference electrode cable 110 located at the middle end 102 of the substrate, a reference electrode contact 112 located at the distal end 103 of the substrate, and the reference electrode layer 113 is provided on the reference electrode cable 110; the counter electrode comprises a counter electrode layer 104 and a counter electrode cable 105 located at the middle end 102 of the substrate, and a counter electrode contact 106 located at the distal end 103 of the substrate.

The sensor electrode structure G comprises a channel 114 located between the first working electrode cable 108 and the reference electrode layer 113, and the first working electrode and the reference electrode are separated by the channel 114; the width of the channel 114 is adjustable within the range of 1µm-280µm, and the length of the channel is adjustable within the range of 30µm-10,000µm. The sensor electrode structure G comprises a channel 114 located between the second working electrode cable 120 and the counter electrode layer 104, and the second working electrode and the counter electrode are separated by the channel 114; the width of the channel 114 is adjustable within the range of 1µm-280µm, and the length of the channel is adjustable within the range of 30µm-10,000µm. The sensor electrode structure G has an L-shape, which includes a first side and a second side, and an angle of 85°-95° is formed at the connection between the first side and the second side. A protruding structure 118 is formed at the outer edge of the connection between the first side and the second side. The sensor electrode structure G comprises a protective layer 116, which is provided outside of the sensor.

FIG. 26 is a structural diagram of the section A-A' of the sensor electrode structure G in Embodiment 7. FIG. 27 is a structural diagram of the section B-B' of the sensor electrode structure G in Embodiment 7. FIG. 28 is a structural diagram of the section C-C' of the sensor electrode structure G in Embodiment 7. It can be seen from FIGs. 26-28 that, the first working electrode and the reference electrode are provided on the first surface of the substrate 100, and spatially separated by a channel, and all components of the first working electrode and the reference electrode are on the same plane; the counter electrode and the second working electrode are provided on the second surface of the substrate 100 opposite to the first surface, and spatially separated by a channel, and all components of the counter electrode and the second working electrode are on the same plane.

### Embodiment 8

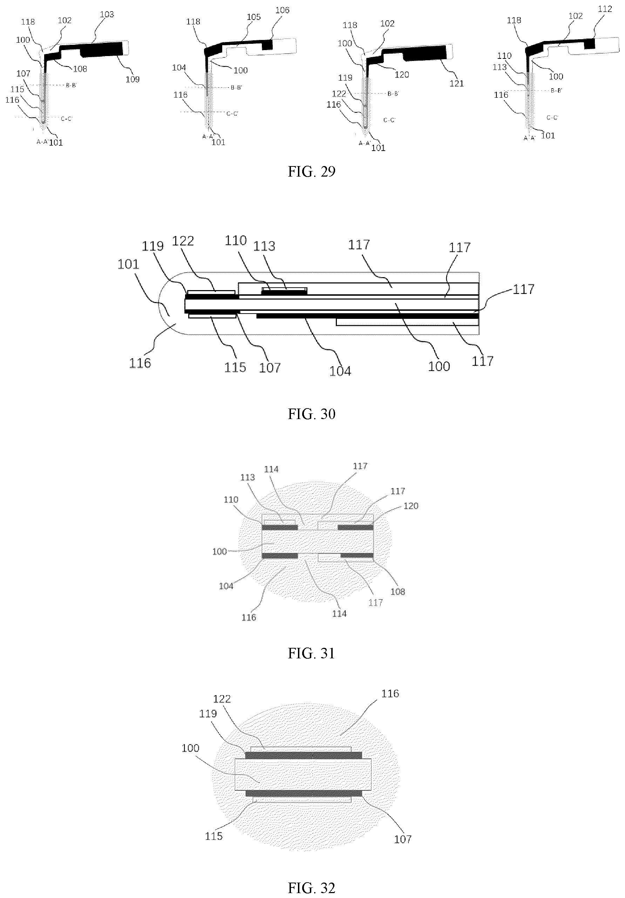

The structure of the sensor electrode structure H in Embodiment 8 is as shown in FIGs. 29-32. FIG. 29 is a view of the sensor electrode structure H in Embodiment 8, where the first left figure is a back view of the second layer of the sensor electrode structure H, and the second left figure is a back view of the first layer of the sensor electrode structure H; the first right figure is a front view of the first layer of the sensor electrode structure H, and the second right figure is a front view of the second layer of the sensor electrode structure H. It can be seen from FIG. 29 that the sensor electrode structure H comprises a substrate 100, a reference electrode, a counter electrode, a first working electrode, and a second working electrode: the substrate 100 has an L-shaped structure and the substrate 100 comprises a front end 101, a middle end 102, and a distal end 103; the counter electrode and the first working electrode are located on a second surface of the substrate 100 opposite to the first surface (i.e., the back of the sensor electrode structure H), and the reference electrode and the second working electrode are located on a first surface of the substrate 100 (i.e., the front of the sensor electrode structure H); specifically, the reference electrode and the second working electrode is located on a first surface of the substrate 100, and spatially separated by a channel, and some components of the reference electrode and the second working electrode are on the same plane, while some components are on different planes; the counter electrode and the first working electrode are located on a second surface of the substrate 100 opposite to the first surface, and spatially separated by a channel, and some components of the counter electrode and the first working electrode are on the same plane, while some components are on different planes.

The first working electrode comprises a first working electrode layer 107 located at the front end 101 of the substrate, a first working electrode cable 108 located at the middle end 102 of the substrate, and a first working electrode contact 109 located at the distal end 103 of the substrate; the first working electrode comprises a first sensing layer 115, which is located on the first working electrode layer 107; the second working electrode comprises a second working electrode layer 119 located at the front end 101 of the substrate, a second working electrode cable 120 located at the middle end 102 of the substrate, and a second working electrode contact 121 located at the distal end 103 of the substrate; the second working electrode comprises a second sensing layer 122, which is located on the second working electrode layer 119; the reference electrode comprises a reference electrode layer 113 and a reference electrode cable 110 located at the middle end 102 of the substrate, a reference electrode contact 112 located at the distal end 103 of the substrate, and the reference electrode layer 113 is provided on the reference electrode cable 110; the counter electrode comprises a counter electrode layer 104 and a counter electrode cable 105 located at the middle end 102 of the substrate, and a counter electrode contact 106 located at the distal end 103 of the substrate.

The sensor electrode structure H comprises a channel 114 located between the first working electrode cable 108 and the counter electrode layer 104, and the first working electrode and the counter electrode are separated by the channel 114; the width of the channel 114 is adjustable within the range of 1µm-280µm, and the length of the channel is adjustable within the range of 30µm-10,000µm. The sensor electrode structure H comprises a channel 114 located between the second working electrode cable 120 and the reference electrode layer 113, and the second working electrode and the reference electrode are separated by the channel 114; the width of the channel 114 is adjustable within the range of 1µm-280µm, and the length of the channel is adjustable within the range of 30µm-10,000µm. The sensor electrode structure H has an L-shape, which includes a first side and a second side, and an angle of 85°-95° is formed at the connection between the first side and the second side. A protruding structure 118 is formed at the outer edge of the connection between the first side and the second side. The sensor electrode structure H comprises a protective layer 116, which is provided outside of the sensor.

FIG. 30 is a structural diagram of the section A-A' of the sensor electrode structure H in Embodiment 8. FIG. 31 is a structural diagram of the section B-B' of the sensor electrode structure H in Embodiment 8. FIG. 32 is a structural diagram of the section C-C' of the sensor electrode structure H in Embodiment 8. It can be seen from FIGs. 29-32 that, the reference electrode and the second working electrode are provided on the first surface of the substrate 100, and spatially separated by a channel; some components of the reference electrode and the second working electrode are on the same plane, while some components are on different planes; the counter electrode and the first working electrode are located on a second surface of the substrate 100 opposite to the first surface, and spatially separated by a channel; some components of the counter electrode and the first working electrode are on the same plane, and some components are on different planes.

### Embodiment 9

The structure of the sensor electrode structure I in Embodiment 9 is as shown in FIGs. 33-36. FIG. 33 is a view of the sensor electrode structure I in Embodiment 8, where the first left figure is a back view of the second layer of the sensor electrode structure I, and the second left figure is a back view of the first layer of the sensor electrode structure I; the first right figure is a front view of the first layer of the sensor electrode structure I, and the second right figure is a front view of the second layer of the sensor electrode structure I. It can be seen from FIG. 33 that the sensor electrode structure I comprises a substrate 100, a reference electrode, a counter electrode, a first working electrode, and a second working electrode: the substrate 100 has an L-shaped structure and the substrate 100 comprises a front end 101, a middle end 102, and a distal end 103; the counter electrode and the reference electrode are located on a second surface of the substrate 100 opposite to the first surface (i.e., the back of the sensor electrode structure I), and the first working electrode and the second working electrode are located on a first surface of the substrate 100 (i.e., the front of the sensor electrode structure I); specifically, the first working electrode and the second working electrode are located on a first surface of the substrate 100, and spatially separated by a channel, and some components of the first working electrode and the second working electrode are on the same plane, while some components are on different planes; the reference electrode and the counter electrode are located on a second surface of the substrate 100 opposite to the first surface, and spatially separated by a channel, and some components of the reference electrode and the counter electrode are on the same plane, while some components are on different planes.

The first working electrode comprises a first working electrode layer 107 and a first working electrode cable 108 located at the middle end 102 of the substrate, and a first working electrode contact 109 located at the distal end 103 of the substrate; the first working electrode comprises a first sensing layer 115, which is located on the first working electrode layer 107; the second working electrode comprises a second working electrode layer 119 located at the front end 101 of the substrate, a second working electrode cable 120 located at the middle end 102 of the substrate, and a second working electrode contact 121 located at the distal end 103 of the substrate; the second working electrode comprises a second sensing layer 122, which is located on the second working electrode layer 119; the reference electrode comprises a reference electrode layer 113 and a reference electrode cable 110 located at the middle end 102 of the substrate, a reference electrode contact 112 located at the distal end 103 of the substrate, and the reference electrode layer 113 is provided on the reference electrode cable 110; the counter electrode comprises a counter electrode layer 104 located at the front end 101 of the substrate, a counter electrode cable 105 located at the middle end 102 of the substrate, and a counter electrode contact 106 located at the distal end 103 of the substrate.

The sensor electrode structure I comprises a channel 114 located between the second working electrode cable 120 and the first working electrode layer 107, and the first working electrode and the second working electrode are separated by the channel 114; the width of the channel 114 is adjustable within the range of 1µm-280µm, and the length of the channel is adjustable within the range of 30µm-10,000µm. The sensor electrode structure I comprises a channel 114 located between the counter electrode cable 105 and the reference electrode layer 113, and the counter electrode and the reference electrode are separated by the channel 114; the width of the channel 114 is adjustable within the range of 1µm-280µm, and the length of the channel is adjustable within the range of 30µm-10,000µm. The sensor electrode structure I has an L-shape, which includes a first side and a second side, and an angle of 85°-95° is formed at the connection between the first side and the second side. A protruding structure 118 is formed at the outer edge of the connection between the first side and the second side. The sensor electrode structure I comprises a protective layer 116, which is provided outside of the sensor.

FIG. 34 is a structural diagram of the section A-A' of the sensor electrode structure I in Embodiment 9. FIG. 35 is a structural diagram of the section B-B' of the sensor electrode structure I in Embodiment 9. FIG. 36 is a structural diagram of the section C-C' of the sensor electrode structure I in Embodiment 9. It can be seen from FIGs. 34-36 that, the first working electrode and the second working electrode are located on a first surface of the substrate 100, and spatially separated by a channel; some components of the first working electrode and the second working electrode are on the same plane, while some components are on different planes; the reference electrode and the counter electrode are located on a second surface of the substrate 100 opposite to the first surface, and spatially separated by a channel; some components of the reference electrode and the counter electrode are on the same plane, while some components are on different planes.

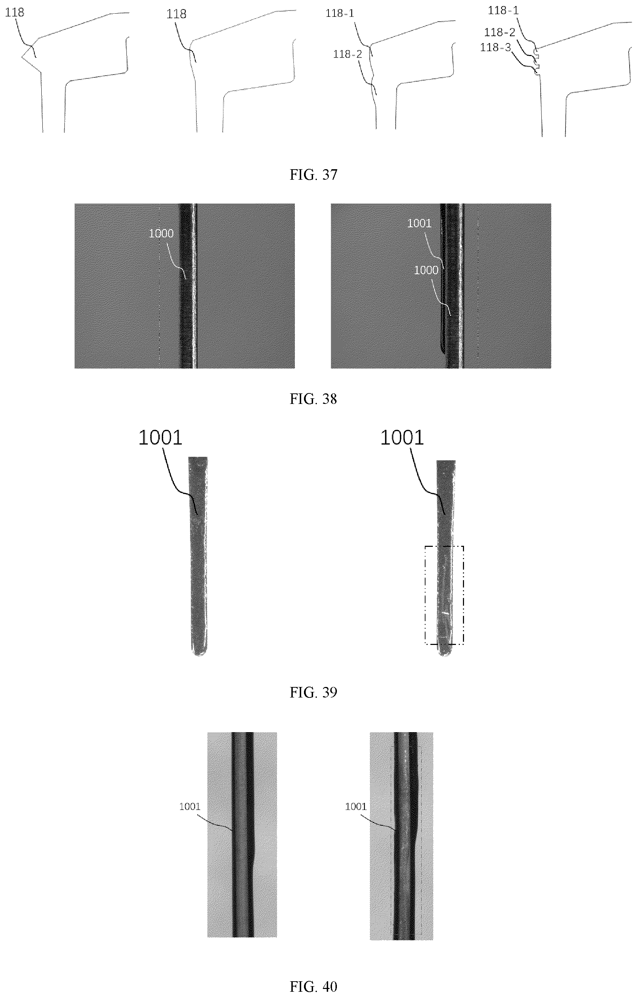

FIG. 38 is a schematic diagram of the combination of the sensor with a protruding structure and the auxiliary device (see left), as well as a schematic diagram of the combination of the sensor without a protruding structure and the auxiliary device (see right), where the reference number 1000 is the auxiliary device, and 1001 is the sensor. It can be seen from FIG. 38 that the sensor without a protruding structure is partially located outside of the auxiliary device.

FIG. 39 is a structural diagram of the sensor surface after implantation of the sensor with the protruding structure into the human body (see left), as well as a structural diagram of the sensor surface after implantation of the sensor without the protruding structure into the human body (see right). It can be seen from FIG. 39 that the sensor without a protruding structure shows damage to the sensor surface after implantation into the human body, while the sensor with a protruding structure does not show any damage to the sensor surface after implantation into the human body.

FIG. 40 is a structural diagram of the sensor after implantation of the sensor with the channel structure into the human body (see left), as well as a structural diagram of the sensor after implantation of the sensor without the channel structure (i.e., stacked structure) into the human body (see right). From FIG. 40, it can be seen that after the sensor without a channel structure (i.e. a stacked structure) is implanted into the human body, "blood stains" can be seen on the side of the sensor. The presence of "blood stains" indicates that there is significant bleeding at the wearing position when the sensor is implanted, which affects the wearing experience from the user's perspective; the sensor with a channel structure has a more suitable thickness, so there are no obvious "blood stains" on the side, indicating that the sensor with a channel structure is worn more smoothly and has a better wearing experience.

### Comparative Example 1

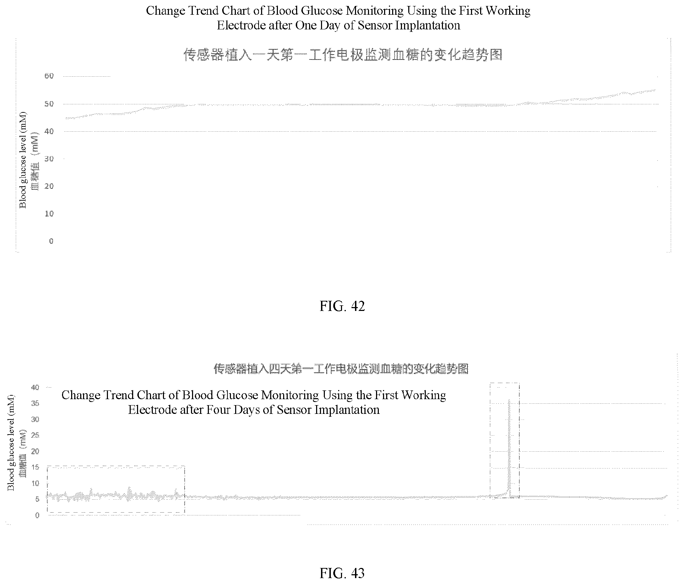

The other structures are as shown in Embodiment 7, with the only difference being that the width of channel 114 in the sensor of Comparative Example 1 is less than 1µm, which can cause the first working electrode cable to come into contact with the reference electrode, and the first working electrode and reference electrode are prone to short circuits. The change trend chart of blood glucose after implanting the sensor into a normal human body for one day is as shown below in FIG. 42. It can be seen from FIG. 42 that due to the narrow channel width in the sensor, the first working electrode and the reference electrode are prone to short circuits, which can lead to high blood glucose levels. Normal blood glucose readings are generally between 3.9mM and 6.1mM.

### Comparative Example 2

The other structures are as shown in Embodiment 7, with the only difference being that the width of channel 114 in the sensor of Comparative Example 2 is greater than 280µm, which can cause the electrode/electrode cable to be too narrow, making it easy to cause an open circuit. The change trend chart of blood glucose after implanting the sensor into a normal human body for four days is as shown below in FIG. 43. It can be seen from FIG. 43 that, due to the wide channel width in the sensor, the first working electrode cable is too narrow, leading to electrode disconnection, which can cause data "jumping" or "jittering" during the blood glucose testing process, affecting the stability of testing data.

The above provides an explanation of the embodiments of the present application. However, the present application is not limited to the above embodiments. Any modification, equivalent replacement and improvement made within the spirit and rule of the present invention shall be incorporated in the scope of protection of the present invention.

## Claims

1. An analyte sensor, comprising a substrate, a first working electrode, a second working electrode, a reference electrode, a counter electrode, a first sensing layer and a second sensing layer; wherein the first sensing layer is provided on the first working electrode, and the second sensing layer is provided on the second working electrode; wherein any two of the first working electrode, the second working electrode, the reference electrode, and the counter electrode are located on a first surface of the substrate, and the remaining two electrodes of the first working electrode, the second working electrode, the reference electrode, and the counter electrode are located on a second surface of the substrate opposite to the first surface;
wherein any two electrodes located on the first surface of the substrate are spatially separated by a channel, and/or, any two electrodes located on the second surface of the substrate opposite to the first surface are spatially separated by a channel;
wherein the width of the channel is 1µm-280µm; the length of the channel is 30µm-10,000µm.

2. The analyte sensor according to claim 1, wherein the first working electrode and the reference electrode are provided on the first surface of the substrate, and the counter electrode and the second working electrode are provided on the second surface of the substrate opposite to the first surface; the first working electrode and the reference electrode are spatially separated by a channel, and/or, the counter electrode and the second working electrode are spatially separated by a channel; or,
wherein the first working electrode and the counter electrode are provided on the first surface of the substrate, and the reference electrode and the second working electrode are provided on the second surface of the substrate opposite to the first surface; the first working electrode and the counter electrode are spatially separated by a channel, and/or, the reference electrode and the second working electrode are spatially separated by a channel; or, wherein the counter electrode and the reference electrode are provided on the first surface of the substrate, and the first working electrode and the second working electrode are provided on the second surface of the substrate opposite to the first surface; the counter electrode and the reference electrode are spatially separated by a channel, and/or, the first working electrode and the second working electrode are spatially separated by a channel;
preferably, any two electrodes located at the same surface of the substrate and spatially separated by a channel are completely on the same plane; or, any two electrodes located at the same surface of the substrate and spatially separated by a channel are partially on the same plane.

3. An analyte sensor, **characterized in that**, the analyte sensor comprises a substrate, a first working electrode, a second working electrode, a reference electrode, a counter electrode, a first sensing layer and a second sensing layer; the first sensing layer is provided on the first working electrode, and the second sensing layer is provided on the second working electrode;
wherein any three of the first working electrode, the second working electrode, the reference electrode, and the counter electrode are located on a first surface of the substrate, and the remaining one electrode of the first working electrode, the second working electrode, the reference electrode, and the counter electrode is located on a second surface of the substrate opposite to the first surface;
wherein one of the three electrodes located on the first surface of the substrate is provided on the surface of the substrate, and the remaining two electrodes are stacked on the surface of the one electrode and separated from the one electrode by a dielectric layer; the remaining two electrodes are spatially separated by a channel;
wherein the width of the channel is 1µm-280µm; the length of the channel is 30µm-10,000µm.

4. The analyte sensor according to claim 3, wherein the first working electrode, the second working electrode, and the reference electrode are provided on the first surface of the substrate, and the counter electrode is provided on the second surface of the substrate opposite to the first surface; or, the first working electrode, the second working electrode, and the counter electrode are provided on the first surface of the substrate, and the reference electrode is provided on the second surface of the substrate opposite to the first surface; or, the first working electrode, the counter electrode, and the reference electrode are provided on the first surface of the substrate, and the second working electrode is provided on the second surface of the substrate opposite to the first surface; or, the second working electrode, the counter electrode, and the reference electrode are provided on the first surface of the substrate, and the first working electrode is provided on the second surface of the substrate opposite to the first surface.
preferably, any two of the three electrodes located at the same surface of the substrate and spatially separated by a channel may be completely on the same plane; or, any two of the three electrodes located at the same surface of the substrate and spatially separated by a channel may be partially on the same plane.

5. The analyte sensor according to any of claims 1-4, wherein the substrate comprises a front end, a middle end, and a distal end, which are sequentially connected; the substrate has an L-shape, which includes a first side and a second side, and an angle of 45°-135° is formed at the connection between the first side and the second side; the front end is located at the first side and is located at the non-connected end of the first side, the distal end is located at the second side and is located at the non-connected end of the second side, and the middle end is located at the first side and the second side, and is located at the connected end of the first side and the connected end of the second side.

6. The analyte sensor according to any of claims 1-5, wherein the first working electrode comprises a first working electrode layer, a first working electrode cable, and a first working electrode contact, and the first working electrode layer is connected to the first working electrode contact through the first working electrode cable; the first working electrode layer is located at the front end of the substrate, the first working electrode cable is located at the middle end of the substrate, and the first working electrode contact is located at the distal end of the substrate; or, the first working electrode layer and the first working electrode cable are located at the middle end of the substrate, and the working electrode contact is located at the distal end of the substrate;
wherein the second working electrode comprises a second working electrode layer, a second working electrode cable, and a second working electrode contact, and the second working electrode layer is connected to the second working electrode contact through the second working electrode cable; the second working electrode layer is located at the front end of the substrate, the second working electrode cable is located at the middle end of the substrate, and the second working electrode contact is located at the distal end of the substrate; or, the second working electrode layer and the second working electrode cable are located at the middle end of the substrate, and the second working electrode contact is located at the distal end of the substrate;
wherein the reference electrode comprises a reference electrode layer, a reference electrode cable, and a reference electrode contact; the reference electrode layer is provided on the reference electrode cable, and the reference electrode cable is connected to the reference electrode contact; the reference electrode layer is located at the front end of the substrate, the reference electrode cable is located at the front end and middle end of the substrate, and the reference electrode contact is located at the distal end of the substrate; or, the reference electrode layer and the reference electrode cable are located at the middle end of the substrate, and the reference electrode contact is located at the distal end of the substrate;
wherein the counter electrode comprises a counter electrode layer, a counter electrode cable, and a counter electrode contact, and the counter electrode layer is connected to the counter electrode contact through the counter electrode cable; the counter electrode layer is located at the front end of the substrate, the counter electrode cable is located at the middle end of the substrate, and the counter electrode contact is located at the distal end of the substrate; or, the counter electrode layer and the counter electrode cable are located at the middle end of the substrate, and the counter electrode contact is located at the distal end of the substrate;
preferably, the counter electrode is configured to form a current closed circuit with the first working electrode, providing a guarantee for converting a first analyte signal into an electrochemical signal, and further configured to form a current closed circuit with the second working electrode, providing a guarantee for converting a second analyte signal into an electrochemical signal;
wherein the reference electrode is configured to form a voltage circuit with the first working electrode, providing a guarantee for converting a first analyte signal into an electrochemical signal, and further configured to form a voltage circuit with the second working electrode, providing a guarantee for converting a second analyte signal into an electrochemical signal.

7. The analyte sensor according to any of claims 1-6, wherein a second sensing layer is provided on the second working electrode layer, and the second working electrode is configured to react with a second analyte; a first sensing layer is provided on the first working electrode layer, and the first working electrode is configured to react with a first analyte;
wherein the thickness of the first working electrode layer is 1µm-20µm; the thickness of the second working electrode layer is 1µm-20µm;
wherein the analyte/physiological indicator that can be monitored by the first sensing layer is blood glucose; the analyte/physiological indicator that can be monitored by the second sensing layer includes one of uric acid, lactic acid, ketones, cholesterol, triglycerides, heart rate, blood pressure, Na⁺, Ka⁺, Ga²⁺, Fe²⁺ and Mg²⁺, etc.

8. The analyte sensor according to any of claims 1-7, wherein any two electrodes spatially separated by a channel refer to that the electrode layer of one electrode and the electrode cable of the other electrode are spatially separated by a channel;
preferably, the width of the channel may be constant, or gradually changing.

9. The analyte sensor according to any of claims 1-8, wherein the analyte sensor has an L-shape, which includes a first side and a second side, and an angle of 45°-135° is formed at the connection between the first side and the second side;
preferably, there are two contact areas and two electrode areas on a first surface of the analyte sensor, and there is one contact area and one electrode area on a second surface of the analyte sensor opposite to the first surface; the electrode area is located at the first side of the L-shaped analyte sensor, and the contact area is located at the second side of the L-shaped analyte sensor; or,
wherein there are two contact areas and two electrode areas on a first surface of the analyte sensor, and there are two contact areas and two electrode areas on a second surface of the analyte sensor opposite to the first surface; or,
wherein there are three contact areas and three electrode areas on a first surface of the analyte sensor, and there is one contact area and one electrode area on a second surface of the analyte sensor opposite to the first surface; the electrode area is located at the first side of the L-shaped analyte sensor, and the contact area is located at the second side of the L-shaped analyte sensor.

10. The analyte sensor according to any of claims 1-9, wherein a protruding structure is formed at the connection between the first side and the second side;
preferably, a protruding structure is formed at the outer edge of the connection between the first side and the second side.
